# EUROPÄISCHE PATENTANMELDUNG

(11) **EP 1 884 564 A1**
(43) Veröffentlichungstag der Anmeldung: **06.02.2008**
(21) Anmeldenummer: 07108967.6
(22) Anmeldetag: 25.05.2007
(51) Int. Cl.: C12M 1/33

(54) **Aufbereitung von Gülle und organischen Feststoffen für eine Fermentation**

(30) Priorität: 01.08.2006 DE 202006011872 U; 14.02.2007 EP 07102358
(71) Anmelder: Eckard, Horst K., 36169 Rasdorf (DE); Grimm, Arnold, 36154 Hosenfeld (DE); Hemschemeier, Hans, 51647 Gummersbach (DE)
(72) Erfinder: Eckard, Horst K., 36169 Rasdorf (DE); Grimm, Arnold, 36154 Hosenfeld (DE); Hemschemeier, Hans, 51647 Gummersbach (DE)
(74) Vertreter: Schwabe - Sandmair - Marx

(57) **Zusammenfassung**

Verfahren zur Aufbereitung von Gülle und organischen Feststoffen für eine Fermentierung, bei dem
a) die organischen Feststoffe mittels einer ersten Zerkleinerungseinrichtung (2; 2, 10; 2, 9; 2, 9, 10) zerkleinert,
f) vor oder nach der Zerkleinerung in der Gülle suspendiert,
g) nach der Zerkleinerung in Suspension mittels einer zweiten Zerkleinerungseinrichtung (10, 11; 11) auf eine größte Erstreckung von maximal 5 mm feiner zerkleinert
h) und in der Suspension gleichmäßig dispergiert werden
i) und die Dispersion direkt oder nach weiterer Behandlung der Fermentierung zugeführt wird.

## Beschreibung

Die Erfindung betrifft ein Verfahren und eine Vorrichtung für die Aufbereitung von Gülle und organischen Feststoffen für eine Fermentation, vorzugsweise für die Erzeugung von Biogas oder Düngern.

Die Nutzung von organischen Rohstoffen durch Fermentation gewinnt an Bedeutung. Entsprechend groß ist das Interesse, Gülle und andere Rohstoffe aus land- und forstwirtschaftlicher Produktion, auch organische Abfälle der Lebensmittelindustrie, Kommunen und privaten Haushalte in Fermentern in Biogas umzuwandeln. Als Nebenprodukt fällt Gärgülle an, die zur Düngung verwendet werden kann. Die Wirtschaftlichkeit heutiger Anlagen ist allerdings noch nicht zufriedenstellend. Die Verweildauer des Ferments im Fermenter ist lang. Da die unterschiedlichen Biofraktionen zur Erzielung des gleichen Abbaugrads unterschiedlich langer Gärzeiten bedürfen, kann eine optimale Verweildauer im Fermenter nur als Kompromiss zwischen einem möglichst vollständigen Abbau und möglichst hohem Durchsatz gewählt oder die Fraktionen müssten getrennt fermentiert werden.

Es ist eine Aufgabe der Erfindung, die Wirtschaftlichkeit von Fermentern, insbesondere in der landwirtschaftlichen oder kommunalen Erzeugung von Biogas, zu erhöhen.

Nach der Erfindung wird aus Gülle und organischen Feststoffen eine Dispersion erzeugt, in der die Feststoffe nur noch als kleine und kleinste Partikel gleichmäßig verteilt sind. Die Partikel haben eine größte Erstreckung von maximal 5 mm, sind also in keiner der drei Raumdimensionen länger als 5 mm. Vorzugsweise werden sie soweit zerkleinert, dass sie eine größte Erstreckung, bezogen auf alle Raumdimensionen, von maximal 2 mm und noch bevorzugter von maximal 1 mm aufweisen.

Die Feststoffe werden in mehreren Zerkleinerungsoperationen mittels einer ersten Zerkleinerungseinrichtung und einer stromabwärts von der ersten Zerkleinerungseinrichtung angeordneten zweiten Zerkleinerungseinrichtung zerkleinert. Die mittels der ersten Zerkleinerungseinrichtung zerkleinerten Feststoffe werden mittels der zweiten Zerkleinerungseinrichtung bis auf die vorstehend genannte Partikelgröße feiner zerkleinert. Die organischen Feststoffe werden der zweiten Zerkleinerungseinrichtung in Suspension mit Gülle zugeführt und in der zweiten Zerkleinerungseinrichtung in Suspension zerkleinert und homogen verteilt. Indem die Feststoffe wenigstens zwei Zerkleinerungsoperationen unterzogen werden und der Zerkleinerungsgrad von Operation zu Operation gesteigert wird, können höhere Zerkleinerungsgrade als bisher erreicht werden. Von Vorteil ist ferner, dass zumindest mit der Operation, in der die geforderte Partikelgröße erreicht wird, die Feststoffpartikel gleichzeitig auch gleichmäßig fein in der Gülle verteilt werden und das Ausgangsprodukt eine quasi homogene Dispersion von Gülle und feinen Feststoffpartikeln ist.

Die organischen Feststoffe können der ersten Zerkleinerungseinrichtung in einem Trockengemisch oder bereits in Suspension mit der Gülle zugeführt werden. Die organischen Feststoffe können in einem heterogenen Gemisch, beispielsweise einem Gemisch aus Energiepflanzen, Heu, Gras und Abfällen landwirtschaftlicher Produktion, aber auch in Form beispielsweise nur einer Pflanzenart zugeführt werden. Die Zerkleinerungseinrichtungen sind in solch einer Weise aufeinander abgestimmt, dass die üblicherweise in der landwirtschaftlichen Produktion anfallenden Feststoffe mittels der ersten Zerkleinerungseinrichtung wenigstens soweit zerkleinert werden, d.h. bis auf eine maximale Partikelgröße, dass sie in Suspension mittels der zweiten Zerkleinerungseinrichtung problemlos feiner zerkleinert werden und die zweite Zerkleinerungseinrichtung nicht verstopfen oder beschädigen können. Indem mehrere Zerkleinerungsoperationen aufeinander aufbauend, d.h. aufeinander abgestimmt ausgeführt werden, kann eine Dispersion, fest in flüssig, erzeugt werden, in der in bevorzugten Ausführungen die größten Partikel am Auslass der zweiten Zerkleinerungseinrichtung eine maximale Erstreckung von höchstens 100 Mikrometern oder wenigen Hundert Mikrometern haben. Die mittels der zweiten Zerkleinerungseinrichtung erzeugte Dispersion weist vorzugsweise eine sahneförmige Konsistenz auf, ist also emulsionsartig.

Die erste oder die zweite Zerkleinerungseinrichtung kann einen einzigen oder mehrere Zerkleinerer aufweisen. Dies schließt auch den Fall ein, dass sowohl die erste als auch die zweite Zerkleinerungseinrichtung mehrere Zerkleinerern umfasst. Das Wort "oder" wird hier und auch sonst stets im üblichen logischen Sinne von "und/oder" verstanden, schließt also die Bedeutung von "entweder... oder" und die Bedeutung von "sowohl als auch" ein, soweit sich aus dem jeweiligen Zusammenhang nicht zwangsläufig etwas anderes ergibt. Weist eine der Zerkleinerungseinrichtung mehrere Zerkleinerer auf, können die Zerkleinerer der jeweiligen Zerkleinerungseinrichtung parallel oder seriell im Strom der zu zerkleinernden Feststoffe oder gemischt parallel und seriell angeordnet sein, was wie gesagt auch eine Anordnung beinhaltet, in der wenigstens zwei Zerkleinerer parallel und wenigstens zwei Zerkleinerer seriell angeordnet sind.

Seriell hintereinander angeordnete Zerkleinerer unterscheiden sich in bevorzugten Ausführungen nicht nur hinsichtlich des Zerkleinerungsgrads am Auslass des jeweiligen Zerkleinerers, sondern auch der Bauart nach und entsprechend in der Wirkungsweise, wie der jeweilige Zerkleinerer auf die organischen Feststoffe wirkt.

Die erste Zerkleinerungseinrichtung ist vorzugsweise so gebildet, dass sie voluminöse Feststoffe bricht, beispielsweise Kartoffeln und Rüben, und lange Zellulosefasern zerreißt sowie aus Fasermaterial gebildete Ballen auseinanderreißt. Die erste Zerkleinerungseinrichtung kann für das Brechen und Zerreißen separat voneinander ein Brechwerk und ein Reißwerk, d.h. für jede dieser Zerkleinerungsarten je wenigstens einen separaten Zerkleinerer umfassen. Bevorzugter weist sie wenigstens ein kombiniertes Brech- und Reißwerk auf, das beide Zerkleinerungsarten gemeinsam in einer Zerkleinerungsoperation ausführt. Ein kombiniertes Brech- und Reißwerk hat den Vorteil, dass faserige und voluminöse Feststoffe, beispielsweise Kartoffeln, Heu, auch Heuballen oder generell faseriges Ballenmaterial, im Gemisch mittels des gleichen Zerkleinerers zerkleinert werden können. Weist die erste Zerkleinerungseinrichtung jedoch mehrere Zerkleinerer unterschiedlicher Bauart auf, werden die Feststoffe vorzugsweise ihrer Art nach den Zerkleinerern separat dem jeweils zugeordneten Zerkleinerer zugeführt und mit der artspezifisch angepassten Zerkleinerungsoperation zerkleinert. Grundsätzlich könnten die Feststoffe aber auch nacheinander durch die unterschiedlichen Zerkleinerer geführt werden.

Die erste Zerkleinerungseinrichtung oder, falls sie mehrere Zerkleinerer umfasst, nur ein einzelner oder ausgewählte mehrere Zerkleinerer der ersten Zerkleinerungseinrichtung kann oder können vorteilhafterweise auch eine Fördereinrichtung für die Feststoffe bilden. Ein Zerkleinerer, der die zu zerkleinernden Feststoffe gleichzeitig auch fördert, umfasst vorzugsweise zwei nebeneinander angeordnete Förderschnecken, die vorzugsweise gegenläufig drehangetrieben werden. Die Förderschnecken können axial durchgehend je einen einzigen Gang mit vorzugsweise mehreren umlaufenden Windungen aufweisen, so dass die organischen Feststoffe längs den zusammenwirkenden Förderschnecken nur in eine Richtung transportiert werden. In einer ebenfalls bevorzugten Ausführung weisen die Förderschnecken axial nebeneinander zwei Windungsgänge auf, vorzugsweise mit je mehreren Windungen, von denen der eine links- und der andere rechtsgängig ist, so dass die Förderschnecken bei einem Drehantrieb von den beiden axialen Enden der Windungsgänge aus zur axialen Mitte fördern. Die Förderschnecken sind so eingerichtet, dass voluminöse Feststoffe während der Förderung gebrochen werden. Vorzugsweise sind die Förderschnecken mit Reißzähnen besetzt, die von der jeweiligen Drehachse weg nach außen abragen und die Feststoffe bei gegenläufigem Drehantrieb in einen zwischen den Förderschnecken gebildeten Spalt ziehen. Auf diese Weise wird ein Brech- und Reißwerkzeug in Kombination mit einer Fördereinrichtung geschaffen.

Die erste Zerkleinerungseinrichtung umfasst einen Grobzerkleinerer, der vorzugsweise in einem Trockenteil der Vorrichtung angeordnet ist und die Feststoffe im trockenen Zustand zerkleinert. Grundsätzlich kann der Grobzerkleinerer jedoch auch in einem Nassteil der Vorrichtung angeordnet sein und dementsprechend die in der Gülle suspendierten Feststoffe zerkleinern. Der Grobzerkleinerer ist in bevorzugten Ausführungen ein im Strom der Feststoffe stromaufwärtigster Zerkleinerer. Er verarbeitet die Feststoffe vorzugsweise in einem Rohzustand, in dem sie aus der Produktion oder als Abfall unmittelbar anfallen, wie beispielsweise Grasschnitt, Heu, Stroh, Futterpflanzen, Energiepflanzen, Silage, Obst und dergleichen. Er zerkleinert die im Rohzustand, vorzugsweise in einem Rohgemisch, vorliegenden Feststoffe so weit, dass der stromabwärts nächste Zerkleinerer sie weiter zerkleinern kann. Vorzugsweise zerkleinert er die Feststoffe so weit, dass sie eine größte Erstreckung von maximal 30 cm, bevorzugter maximal 20 cm aufweisen.

Die erste oder die zweite Zerkleinerungseinrichtung umfasst in bevorzugten Ausführungen einen Feinzerkleinerer, der die organischen Feststoffe trocken oder vorzugsweise in Suspension bis auf eine größte Erstreckung von maximal 5 cm, bevorzugter maximal 2 cm zerkleinert. Der Feinzerkleinerer zerschneidet die Feststoffe, vorzugsweise zerreißt er sie auch. Er kann ferner so ausgebildet sein, dass er sie auch zerdrückt.

Der Feinzerkleinerer kann drehantreibbare Messerwellen aufweisen, vorzugsweise zwei gegenläufig drehangetriebene Messerwellen, mit um die Drehachse der jeweiligen Messerwelle umlaufenden Schneidzähnen. Jede der Messerwellen weist Gruppen von Schneidzähnen auf, die voneinander axial beabstandet längs der jeweiligen Drehachse angeordnet sind. Die Schneidzähne jeweils einer Gruppe sind auf zumindest im Wesentlichen gleicher axialen Höhe in Umfangsrichtung voneinander beabstandet um die Drehachse der jeweiligen Messerwelle verteilt angeordnet. Die Messerwellen sind zueinander vorzugsweise außenachsig angeordnet. Sie greifen wie zwei Kämme ineinander. Die Feststoffe werden durch einen zwischen den Messerwellen verbleibenden Zickzack-Spalt gefördert und dabei zerschnitten. Um die Feststoffe ferner auch zu zerreißen, sind die Schneidzähne vorzugsweise so geformt, dass sie mit Feststoffen verhaken. So können sie beispielsweise als Sägezähne geformt sein, deren steile Kante vorlaufend angeordnet ist. Die Messerwellen werden vorzugsweise je mit solch einer Umfangsgeschwindigkeit angetrieben, dass die Schneidzähne der einen Messerwelle im Eingriffsbereich eine größere Umfangsgeschwindigkeit als die Schneidzähne der anderen Messerwelle haben.

In bevorzugten Ausführungen umfasst die zweite Zerkleinerungseinrichtung einen Homogenisierer der den Feinzerkleinerer bilden oder für eine Feinstzerkleinerung als dritte Stufe der Zerkleinerung stromabwärts von einem Feinzerkleinerer angeordnet sein kann. Mit Vorteil kann ein Homogenisierer verwendet werden, wie er in der Herstellung von Pasten und Cremes in der pharmazeutischen Industrie bereits verwendet wird. Der Homogenisierer zerkleinert die vorzerkleinerten Feststoffe mittels Schneiden oder Kavitation, vorzugsweise mittels einer Kombination beider Zerkleinerungsoperationen.

Der Homogenisierer umfasst vorzugsweise wenigstens eine Zerkleinerungsstufe aus einem ersten Scherkranz, einem zweiten Scherkranz und einem Drehantrieb für den ersten Scherkranz. Der erste Scherkranz bildet einen Rotor. Der zweite Scherkranz bildet einen Stator, der vorzugsweise still steht und verdrehgesichert mit einem Gehäuse des Homogenisierers verbunden ist. Grundsätzlich kann jedoch zusätzlich auch der zweite Scherkranz relativ zu dem Gehäuse drehangetrieben sein, vorzugsweise gegenläufig zum ersten Scherkranz. Der erste Scherkranz weist in Umfangsrichtung voneinander beabstandete Scherkanten auf, die quer zu der Umfangsrichtung des Scherkranzes weisen. Der zweite Scherkranz weist ebenfalls in Umfangsrichtung um die Rotationsachse voneinander beabstandete Scherkanten auf, die Schergegenkanten für die Scherkanten des ersten Scherkranzes bilden und quer zu der Umfangsrichtung des zweiten Scherkranzes weisen. Die Scherkanten und Schergegenkanten erstrecken sich vorzugsweise parallel zueinander und vorzugsweise parallel zu der Rotationsachse des zweiten Scherkranzes. Grundsätzlich könnten Sie zu der Rotationsachse jedoch auch geneigt sein, solange sie nur eine quer zu der Umfangsrichtung weisende Richtungskomponente haben. Die Scherkränze sind relativ zueinander vorzugsweise innenachsig angeordnet, so dass der eine den anderen umgibt.

Zwischen den beiden Scherkränzen der Zerkleinerungsstufe verbleibt um die Rotationsachse herum ein enger Spalt, in den die Suspension einleitbar ist. In dem Spalt werden die Feststoffe mittels der relativ zueinander rotierenden Scherkanten und Schergegenkanten zerkleinert, wobei die Zerkleinerung zumindest zu einem Teil auf einer Scherwirkung der relativ zueinander bewegten Scher- und Schergegenkanten beruht. Von besonderem Vorteil sind die Druckpulsationen, die mit jedem Schervorgang einhergehen. Die Drehgeschwindigkeit, welche die beiden Scherkränze relativ zueinander aufweisen, ist vorteilhafterweise so groß, dass die Feststoffe über die Scherwirkung hinaus auch durch Kavitation zerkleinert werden. Vorzugsweise ist die Geschwindigkeit so groß, dass die Kavitation an der Zerkleinerung einen in etwa ebenso großen oder vorteilhafterweise sogar größeren Anteil als die Scherwirkung hat. Um Kavitation in einem nennenswerten Ausmaß zu erzeugen, wird der Homogenisierer mit solch einer Geschwindigkeit drehangetrieben, dass radial nächstbenachbarte Scherkränze am Umfang eine Relativgeschwindigkeit von wenigstens 30 m/s, vorzugsweise wenigstens 50 m/s und noch bevorzugter wenigstens 60 m/s zueinander haben.

Die Suspension kann grundsätzlich zwar in axialer Richtung in den Spalt eingebracht werden, bevorzugter strömt sie jedoch durch einen der Scherkränze in den Spalt und in einer Weiterentwicklung durch den anderen der beiden Scherkränze auch wieder aus dem Spalt. In derartigen Ausführungen ist wenigstens einer der Scherkränze, vorzugsweise sind beide Scherkränze, radial zur Rotationsachse durchlässig für die Suspension. Es würde hierfür genügen, wenn nur zwischen zwei benachbarten Scherkanten oder Schergegenkanten im jeweiligen Scherkranz ein einziger Durchlass geformt ist, bevorzugter ist ein Durchlass jedoch zwischen je zwei nächstbenachbarten Scherkanten oder Schergegenkanten vorhanden.

Der Homogenisierer umfasst in bevorzugten Weiterentwicklungen wenigstens eine, bevorzugter mehrere, weitere Zerkleinerungsstufen. Die Suspension wird sukzessive durch die mehreren Zerkleinerungsstufen gefördert und von Stufe zu Stufe feiner zerkleinert. Die Serienschaltung kann dergestalt sein, dass die mehreren Zerkleinerungsstufen in Bezug auf die Rotationsachse entweder nur radial oder nur axial hintereinander angeordnet sind. Bevorzugter sind wenigstens zwei Zerkleinerungsstufen in der Art der erläuterten Zerkleinerungsstufe radial hintereinander und auch wenigstens zwei derartige Zerkleinerungsstufen axial hintereinander angeordnet, so dass die durch den Homogenisierer geförderte Suspension wenigstens durch zwei oder drei Zerkleinerungsstufen gefördert wird. Die Breite des Spalts oder die Anzahl der Scherkanten oder Schergegenkanten kann von Stufe zu Stufe variieren, vorzugsweise so, dass die Spaltbreite sich von Stufe zu Stufe in Förderrichtung verringert oder die Anzahl der Kanten sich in Förderrichtung von Stufe zu Stufe vergrößert, was auch den Fall einer Kombination beider Variationsmöglichkeiten einschließt. Die Verringerung der Spaltbreite oder die Vergrößerung der Anzahl der Kanten muss auch nicht bei jeder der Zerkleinerungsstufen auf die jeweils nächste vorgenommen werden, grundsätzlich genügt es, wenn eine Verringerung der Spaltbreite oder eine Vergrößerung der Anzahl der Scher- oder Schergegenkanten nur einmal zwischen zwei aufeinander folgenden Stufen stattfindet. Eine Variation der genannten Art zwischen mehr als nur zwei der Zerkleinerungsstufen wird jedoch bevorzugt.

Der Homogenisierer ist vorteilhaft zwar insbesondere in Kombination mit der beanspruchten Erfindung, nämlich der Zerkleinerung in mehreren Stufen, er ist jedoch auch als solcher vorteilhaft für die Aufbereitung von Biomasse zum Zwecke der Biogaserzeugung aus den genannten Ausgangsstoffen, insbesondere einer Suspension fester Biobestandteile in einer Flüssigkeit, wie beispielsweise faseriger Feststoffe in Gülle, Wasser oder einem Gemisch derartiger Flüssigkeiten.

Obgleich die Vorrichtung entweder nur den Feinzerkleinerer oder den Homogenisierer umfassen kann, entspricht es bevorzugten Ausführungen, wenn sowohl der Feinzerkleinerer als auch der Homogenisierer vorgesehen ist. Sind beide Zerkleinerer vorhanden, unterscheiden sie sich im Ergebnis der jeweiligen Behandlung. Die vom Homogenisierer behandelten Feststoffe weisen einen höheren Zerkleinerungsgrad als die vom Feinzerkleinerer behandelten Feststoffe auf. Allerdings wird dem Homogenisierer vorteilhafterweise nur Biomasse mit größten Festbestandteilen zugeführt, die kleiner sind als die größten Festbestandteile in der dem Feinzerkleinerer zugeführten Biomasse. Der Homogenisierer ist dem Feinzerkleinerer in bevorzugten Ausführungen nachgeschaltet, so dass die bereits im Feinzerkleinerer zerkleinerten Feststoffe im Homogenisierer feiner zerkleinert werden. Vorzugsweise ist der Homogenisierer dazu eingerichtet, die ihm zugeführte Biomasse soweit zu zerkleinern, dass den Homogenisierer eine homogene, fließfähige Biomasse mit größten Partikeln von höchsten 5 mm, vorzugsweise höchstens 2 mm, verlässt. Noch bevorzugter ist der Homogenisierer so ausgebildet, dass die größten Partikel am Auslass des Homogenisierers eine maximale Erstreckung von höchstens 100 Mikrometern oder wenigen hundert Mikrometern haben. Die mittels des Homogenisierers dispergierte Biomasse weist vorteilhafterweise eine sahneförmige Konsistenz auf, d.h. sie ist emulsionsartig. Mit dem Homogenisierer gelingt insbesondere die in herkömmlichen Aufbereitungsanlagen problematische Zerkleinerung von zellulosehaltigem Fasermaterial auf maximale Längen aus dem unteren Millimeterbereich bis in den Submillimeterbereich.

Ist sowohl der Feinzerkleinerer als auch der Homogenisierer vorgesehen, ist es vorteilhaft, wenn der Homogenisierer stromabwärts von dem Feinzerkleinerer angeordnet ist. Die beiden Komponenten sind vorzugsweise derart aufeinander abgestimmt, dass der Feinzerkleinerer eine Zerkleinerung der Feststoffbestandteile auf eine maximale Erstreckung sicherstellt, die nicht größer ist als ein größter lichter Abstand, den in Umfangsrichtung nächstbenachbarte Scherkanten des gleichen Scherkranzes oder in radialer Richtung die Scherkanten und Schergegenkanten radial nächstbenachbarter Scherkränze des Homogenisierers voneinander haben. Der Abstand in Umfangsrichtung wird an einem Scherkranz und der Abstand in radialer Richtung zwischen zwei Scherkränzen der in Strömungsrichtung ersten Scherstufe, d. h. der Eingangsstufe des Homogenisierers gemessen.

Die erfindungsgemäß aufbereitete Biomasse enthält nachwachsende Rohstoffe aus land- oder forstwirtschaftlicher Produktion. Sie kann pflanzliche oder tierische Abfallprodukte enthalten, auch Speisereste oder Schlachtabfälle. Sie kann frische biologische Produkte, beispielsweise Gras, Futter- oder Energiepflanzen, oder Silage, Heu oder Stroh enthalten. Klärschlamm kann ebenfalls beigemischt werden. Die Ausgangsstoffe oder -fraktionen können einzeln oder vorteilhafterweise auch in beliebiger Kombination Inhaltsstoffe der Dispersion sein, die mittels der Vorrichtung erzeugt wird. Aufgrund der feinen Dispergierung der Festbestandteile wird die Geschwindigkeit der Vergärung im Fermenter erhöht und die erforderliche Verweildauer verringert. Sind die Feststoffpartikel bis auf eine größte Erstreckung von höchstens 2 mm oder vorzugsweise noch weiter zerkleinert, nähern sich die Kennlinien der Vergärung unterschiedlichster Pflanzen, beispielsweise von frischem Mais und Gras, einander stark an. Gemeint sind die Kennlinien m(t), d. h. die aus dem jeweiligen Inhaltsstoff, also einer bestimmten Pflanzensorte in einem bestimmten Zustand, beispielsweise frisch, getrocknet oder siliert, durch Vergärung erzeugte Gasmenge über der Verweildauer im Fermenter. Andererseits dienen die nach wie vor vorhandenen kleinen, gleichmäßig fein verteilten Feststoffpartikel den Mikroorganismen im Fermenter als Halt, so dass in den Fermenter keine zusätzlichen Stützstrukturen für die Mikroorganismen eingebracht werden müssen. Die erfindungsgemäße Dispersion kann in kurzer Zeit und in der Dispersion enthaltene, der Art nach unterschiedliche Biofraktionen können wegen der Angleichung der Vergärungskennlinien vollständiger als bislang gemeinsam in einem Fermenter abgebaut werden. Darüberhinaus kann mit der Gärgülle, die durch Cofermentierung von Frischgülle oder Festmist mit den anderen Ausgangsstoffen der erfindungsgemäßen Dispersion entsteht, ein hochwertiger Dünger erzeugt werden.

Falls die Suspension am Auslass des Feinzerkleinerers nur noch Feststoffpartikel mit einer maximalen Größe aufweist, die eine weitgehende Angleichung der genannten Kennlinien m(t) der unterschiedlichen Inhaltsstoffe dieser Biomasse gewährleistet, kann auf den Homogenisierer verzichtet werden. Diese größte Erstreckung der Partikel sollte 4 mm, besser 2 mm und noch besser 1 mm, nicht überschreiten. Falls der Homogenisierer wie bevorzugt zusätzlich zu dem Feinzerkleinerer vorgesehen ist, kann die Partikelgröße in dem vom Feinzerkleinerer behandelten Strom der Biomasse durch Messung im Strom direkt oder auf der Basis beispielsweise der für den Feinzerkleinerer erforderlichen Antriebsleistung oder einer stromauf oder stromab von dem Feinzerkleinerer angeordneten Fördereinrichtung indirekt ermittelt werden, um in Abhängigkeit von der ermittelten maximalen Erstreckung der Partikel die im Feinzerkleinerer behandelte Biomasse wahlweise entweder durch den Homogenisierer zu führen oder an diesem vorbei zu einem Auslass der Vorrichtung zu leiten.

Organische Feststoffe und Gülle können stromaufwärts von der ersten Zerkleinerungseinrichtung und somit stromaufwärts von dem Grobzerkleinerer miteinander gemischt werden. Bevorzugt werden dem Grobzerkleinerer jedoch wie gesagt organische Feststoffe zumindest im Wesentlichen im trockenen Zustand zugeführt und erst die mittels des Grobzerkleinerers zerkleinerten Feststoffe mit der Gülle vermischt. Die Einmischung bzw. Suspendierung dieser Feststoffe kann stromabwärts von dem Feinzerkleinerer stattfinden, falls dem Feinzerkleinerer noch ein Homogenisierer als Feinstzerkleinerer nachgeschaltet ist. Bevorzugter werden die mittels des Grobzerkleinerers vorzerkleinerten Feststoffe jedoch stromaufwärts von dem Feinzerkleinerer in die Gülle eingeleitet und bereits dem Feinzerkleinerer in Suspension zugeführt.

Die Vorrichtung ist mit einer Mischeinrichtung ausgestattet, mittels der die Gülle und die Feststoffe miteinander vermischt werden. Die Mischeinrichtung kann von einem Rohrleitungsstück mit einem Einlass für die Gülle, einem weiteren Einlass für die organischen Feststoffe und einem Auslass für die Suspension gebildet werden. Die Mischeinrichtung kann auch eine Fördereinrichtung oder selbst ein Zerkleinerer sein. Sie kann zu einem Mischbehälter weitergebildet sein, der wenigstens einen Einlass für Gülle, wenigstens einen Einlass für organische Feststoffe und wenigstens einen Auslass für ein Flüssig-Fest-Gemisch von Gülle und organischen Feststoffen aufweist. Ein Mischbehälter ist besonders vorteilhaft, falls Suspension nicht nur in Richtung auf einen Auslass der Vorrichtung, sondern zwecks Einstellung der Viskosität auch zurückgeführt geführt werden soll. In einer bevorzugten ersten Variante wird Gülle, wie sie in landwirtschaftlichen Betrieben anfällt, direkt in den Mischbehälter eingeleitet. In einer alternativen zweiten Variante ist die Mischeinrichtung einem Mischbehälter vorgeschaltet, und es wird bereits eine mittels der Mischeinrichtung gebildete Suspension in den Mischbehälter geleitet. Der Feinzerkleinerer kann stromaufwärts von solch einem Mischbehälter angeordnet sein. In beiden Varianten, insbesondere in der ersten Variante, kann der Feinzerkleinerer stattdessen mit Vorteil stromabwärts von dem Mischbehälter angeordnet sein und dem Feinzerkleinerer die Suspension aus dem Mischbehälter zugeführt werden.

Falls ein Feinzerkleinerer und zusätzlich ein Homogenisierer für eine Feinstzerkleinerung und Dispergierung vorgesehen sind, zweigt zwischen dem Feinzerkleinerer und dem Homogenisierer vorzugsweise eine Rückführleitung ab, durch die im Feinzerkleinerer behandelte Suspension rückführbar ist, vorzugsweise in den Mischbehälter. In einer Verbindungsleitung zwischen dem Feinzerkleinerer und dem Homogenisierer und in der Rückführleitung kann jeweils ein Steuerventil oder im Bereich der Abzweigung ein Mehrwege-Steuerventil vorgesehen sein, um die im Feinzerkleinerer behandelte Suspension wahlweise entweder zum Homogenisierer oder zurück zu führen. In einer Weiterentwicklung solch einer Steuerungseinrichtung können mittels des Mehrwege-Steuerventils oder der mehreren Steuerventile auch Teilströme eingestellt werden, so dass ein dosierter erster Teilstrom zum Homogenisierer und ein zweiter Teilstrom zurückgeführt werden kann. Verfügt die Vorrichtung wie bevorzugt über den Homogenisierer, kann von einer Leitung, die vom Homogenisierer zum Auslass der Vorrichtung führt, eine Rückführleitung abzweigen, um zumindest einen Teil der vom Homogenisierer erzeugten Dispersion zurückführen zu können, beispielsweise in den Mischbehälter. Die Rückführung einer Flüssig-Fest-Mischung mit bereits zerkleinerten Feststoffen erhöht die Flexibilität hinsichtlich der Einstellung der Viskosität der Suspension bzw. Dispersion.

Eine Zuführung für organische Feststoffe umfasst vorzugsweise einen Behälter für die Aufnahme und Zwischenspeicherung der Feststoffe, der im folgenden auch als Vorlagebehälter bezeichnet wird. Der Grobzerkleinerer, der vorzugsweise ein kombiniertes Brech- und Reißwerkzeug und noch bevorzugter ein gleichzeitig auch als Fördereinrichtung gebildetes Brech- und Reißwerkzeug ist, kann vorteilhafterweise in einem unteren Bereich des Vorlagebehälters angeordnet sein. Gegebenenfalls ist im unteren Bereich des Vorlagebehälters auch nur eine Fördereinrichtung für die Feststoffe angeordnet. Der Vorlagebehälter sollte so groß sein, dass eine Füllung wenigstens dem Tagesbedarf eines angeschlossenen oder anzuschließenden Fermenters entspricht.

Vorzugsweise verjüngt sich der Vorlagebehälter in Richtung auf den Grobzerkleinerer oder die reine Fördereinrichtung trichterförmig, vorzugsweise mit geraden, unter einem spitzen Winkel zueinander weisenden Behälterwänden. In derartigen Ausführungen kann der Behälter im unteren Bereich im Querschnitt einen abgestumpften Konus bilden. Die Neigung der Behälterwand oder der mehreren Wände sollte zur Vertikalen nicht größer als 30° sein, um zu verhindern, dass die Feststoffe sich an der Behälterwand festsetzen können. Neigungswinkel von 20° oder weniger werden bevorzugt. Der Grobzerkleinerer oder die reine Fördereinrichtung kann noch innerhalb des Behälters angeordnet sein, was eine Anordnung in einem Auslassquerschnitt des Behälters einschließt. Sie kann stattdessen auch nahe des Auslasses unterhalb des Behälters angeordnet sein.

In einer Weiterentwicklung umfasst die Vorrichtung auch eine Einrichtung zur Behandlung von Biomasse, die tierische Fette enthält. Die Einrichtung arbeitet vorzugsweise separat von den bereits erläuterten anderen Komponenten der Vorrichtung, könnte grundsätzlich aber auch in einem Kreislauf für die Behandlung der weniger kritischen Biomasse integriert sein, falls ein solcher Kreislauf vorhanden ist. Die Einrichtung für die Behandlung der tierischen Fette enthaltenden Biomasse umfasst in bevorzugter Ausführung einen eigenen Zerkleinerer für diese Fraktion. Der Zerkleinerer ist vorzugsweise ein Ultraschall-Zerkleinerer. Vorteilhafte Ultraschall-Zerkleinerer werden in der DE 10 2005 030 895 A1 und der EP 07102358.4 offenbart. Die Einrichtung zur Behandlung der tierische Fette enthaltenden Biomasse umfasst in vorteilhaften Ausführungen ferner einen Sammelbehälter für diese Biomasse und eine eigene Förder- und Dosiereinrichtung, mittels der die Biomasse aus dem Sammelbehälter in und durch den Zerkleinerer förderbar ist.

Die Vorrichtung oder nur bestimmte Komponenten der Vorrichtung können mit Vorteil gemeinsam auf einer Plattform angeordnet sein, die als Einheit auf der Straße oder Schiene transportiert werden kann und entsprechende Abmessungen aufweist. Die Plattform mit den darauf angeordneten Komponenten hat vorteilhafterweise eine Breite von höchstens 3m, bevorzugter höchstens 2,5m, und eine Höhe von vorzgusweise höchstens 5m, vorzugsweise höchstens 4m. Die Länge kann größer sein. Insgesamt kann die Plattform mit den darauf angeordneten Komponenten Abmessungen aufweisen, die den Abmessungen eines Containers entsprechen, der auf einem Eisenbahnwagon oder einem Sattelschlepper transportiert werden kann. Die Plattform ist in einer Weiterentwicklung ein Container, in dem die betreffenden Komponenten aufgenommen sind. Eine derartige Anordnung ist Gegenstand der europäischen Patentanmeldung Nr. 07102358.4, die ergänzend in Bezug genommen wird.

Die Plattform oder vorzugsweise der Plattform-Container weist einen Einlass für Gülle oder gegebenenfalls eine bereits außerhalb der Plattform aus Gülle und organischen Feststoffen gebildete Suspension und ferner einen Auslass für die erzeugte Dispersion auf. Ferner sind auf der Plattform wenigstens zwei der folgenden Komponenten angeordnet:
- der Grobzerkleinerer,
- der Feinzerkleinerer,
- der Homogenisierer,
- wenigstens eine Fördereinrichtung,
- der Vorlagebehälter,
- der Mischbehälter,
- der Ultraschall-Zerkleinerer.

Bevorzugter sind mehr als zwei dieser Komponenten auf der Plattform angeordnet und besonders bevorzugt sind alle genannten Komponenten auf der Plattform angeordnet.

Weitere vorteilhafte Merkmale der Erfindung werden auch in den Unteransprüchen und deren Kombinationen beschrieben.

Nachfolgend werden Ausführungsbeispiele der Erfindung anhand von Figuren erläutert. An den Ausführungsbeispielen offenbar werdende Merkmale bilden je einzeln und in jeder Kombination die Gegenstände der Ansprüche und die vorstehend beschriebenen Ausgestaltungen weiter. Es zeigen:
- Fig. 1: eine Vorrichtung eines ersten Ausführungsbeispiels,
- Fig. 2: eine Vorrichtung eines zweiten Ausführungsbeispiels,
- Fig. 3: eine Container-Plattform und darin aufgenommene Komponenten der Vorrichtung,
- Fig. 4: einen Vorlagebehälter mit Grobzerkleinerer
- Fig. 5: einen Feinzerkleinerer in einem Schnitt,
- Fig. 6: den Feinzerkleinerer in einer perspektivischen Sicht
- Fig. 7: einen Homogenisierer,
- Fig. 8: ein Detail eines Scherwerks des Homogenisierers,
- Fig. 9-11: jeweils einen Ultraschall-Zerkleinerer mit piezokeramischen Schwingelementen zur Aufnahme von unterschiedlichen Ansteuerungsfrequenzen und
- Fig. 12: ein Flussdiagramm einer Zerkleinerung und Dispergierung in mehreren Stufen.

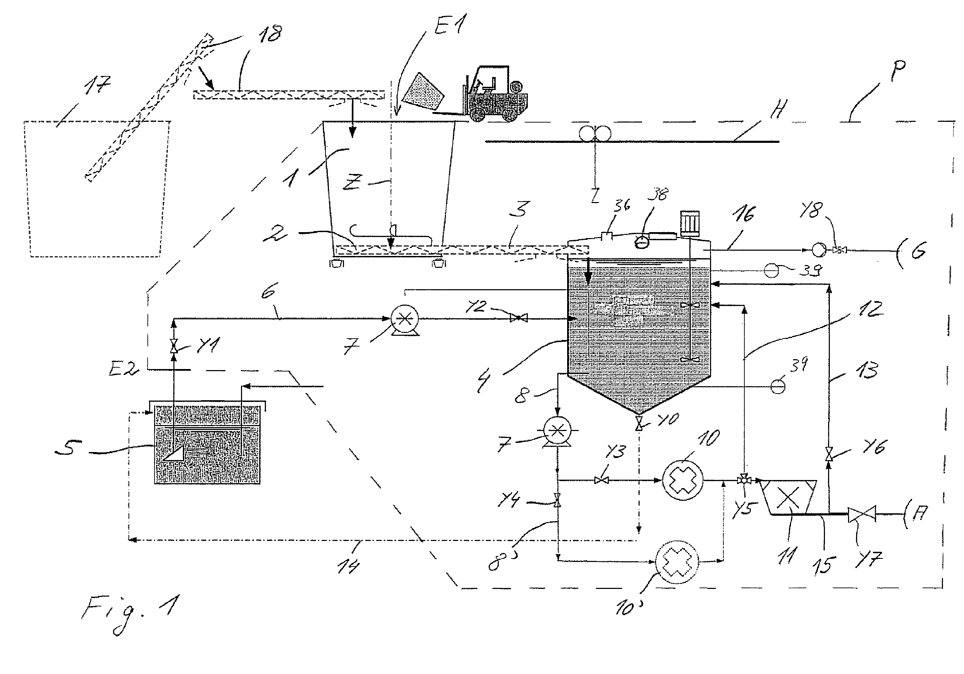

Figur 1 zeigt in einem Verfahrensfließbild eine erfindungsgemäße Vorrichtung in einem ersten Ausführungsbeispiel. Die Vorrichtung umfasst einen Vorlagebehälter 1 für organische Feststoffe, vorzugsweise für die Aufnahme eines Trockengemisches organischer Feststoffe aus landwirtschaftlicher oder forstwirtschaftlicher Produktion. Der Vorlagebehälter 1 ist von oben durch einen Einlass E1 mit den Feststoffen befüllbar, was in Figur 1 durch das symbolisiert dargestellte Förderzeug angedeutet werden soll. In einem unteren Bereich des Vorlagebehälters 1 ist ein Grobzerkleinerer 2 angeordnet, der die Feststoffe zerkleinert und bei der Zerkleinerung aus dem Vorlagebehälter 1 über einen Zwischenförderer 3 in einen Mischbehälter 4 fördert. Die zerkleinerten Feststoffe werden in einen oberen Bereich des Mischbehälters 4 eingeleitet.

Die Vorrichtung weist eine Zuführung für Gülle auf. Die Zuführung umfasst eine Zuführleitung 6 mit einem an ihrem stromaufwärtigen Ende angeordneten Einlass E2 für die Einleitung der Gülle. Die Zuführleitung 6 ist über den als Anschluss gebildeten Einlass E2 an eine mit Gülle gefüllte Vorgrube 5 angeschlossen. In der Zuführleitung 6 ist eine Fördereinrichtung 7 in Form einer Pumpe, beispielsweise einer Exzenterschneckenpumpe, angeordnet, um die Gülle, die mit anderen einer Fermentation zuführbaren Flüssigfraktionen vermischt sein kann, in den Mischbehälter 4 zu fördern. In der Zuführleitung 6 sind Ventile Y1 und Y2 angeordnet, das Ventil Y1 zwischen dem Einlass E2 und der Fördereinrichtung 7 und das Ventil Y2 zwischen der Fördereinrichtung 7 und dem Mischbehälter 4. Die Ventile Y1 und Y2 können einfache Absperrventile oder Dosierventile sein.

Im Mischbehälter 4 werden die Gülle und die bereits vorzerkleinerten organischen Feststoffe vermischt. Der Mischbehälter 4 dient ferner als Massen-Puffer und auch als Mischeinrichtung für weitere Fraktionen der aus der Gülle und den organischen Feststoffen im Mischbehälter gebildeten Suspension. Im Mischbehälter 4 kann wie angedeutet ein Rührwerk angeordnet sein, um einer Sedimentation der Feststoffe entgegenwirken zu können. Der Mischbehälter 4 weist einen Auslass für eine Sumpfentleerung auf, zweckmäßigerweise wie angedeutet am tiefsten Punkt des Mischbehälters 4. Der Auslass für die Sumpfentleerung kann mittels eines Ventils Y0 automatisch wahlweise geschlossen oder geöffnet werden. Vom Auslass führt eine Rückführleitung 14 die Biomasse des Sumpfs zurück zur Vorgrube 5.

Suspension wird aus dem Mischbehälter 4 von oberhalb des Sumpfs über eine Leitung 8 zu einem Feinzerkleinerer 10 geführt. Die Feststoffe werden mittels des Feinzerkleinerers 10 fein zerkleinert und einem Homogenisierer 11 zugeführt. Mittels des Homogenisierers werden die Feststoffe nochmals feiner zerkleinert, d.h. feinst zerkleinert, und bei der Zerkleinerung gleichzeitig homogen im Flüssiganteil der Suspension dispergiert, so dass eine Dispersion sahneförmiger Konsistenz den Homogenisierer 11 an dessen Auslass verlässt. Die Dispersion wird über eine Auslassleitung 15 und einen stromabwärtigen Auslass A der Vorrichtung abgefördert. Der Auslass A ist an einen Fermenter oder eine Zuführleitung für einen Fermenter anschließbar bzw. im Betrieb der Vorrichtung angeschlossen. Alternativ kann die Dispersion zentral erzeugt und in Tankfahrzeugen zu dezentralen Fermentern transportiert werden.

In der vom Mischbehälter 4 zum Feinzerkleinerer 10 führenden Leitung 8 ist eine weitere Fördereinrichtung 7 in Form einer Pumpe, beispielsweise einer Exzenterschneckenpumpe, angeordnet, die einen Förderdruck für den Feinzerkleinerer 10 und im Ausführungsbeispiel auch für den Homogenisierer 11 erzeugt. Gegebenenfalls ist eine weitere Fördereinrichtung zwischen dem Feinzerkleinerer 10 und dem Homogenisierer 11 oder stromabwärts von dem Homogenisierer 11 angeordnet, um einen für diese beiden Zerkleinerer erforderlichen Förderdruck zu erzeugen.

In der Verbindungsleitung, die von dem Feinzerkleinerer 10 zum Homogenisierer 11 führt, ist ein Mehrwege-Steuerventil Y5 angeordnet, bei dem eine Rückführleitung 12 abzweigt, die in den Mischbehälter 4 zurückführt. Über das Steuerventil Y5 kann die Suspension vom Feinzerkleinerer 10 wahlweise entweder zum Homogenisierer 11 oder zurück in den Mischbehälter 4 geführt werden. Mittels des Steuerventils Y5 kann ferner der durch den Feinzerkleinerer 10 geförderte Strom der Suspension in Teilströme aufgespalten werden, von denen der eine zum Homogenisierer 11 und der andere zurück in den Mischbehälter 4 geführt wird. Eine weitere Rückführleitung 13 zweigt stromabwärts von dem Homogenisierer 11 von der Ausgangsleitung 15 ab und führt ebenfalls zurück in den Mischbehälter 4. In der Rückführleitung 13 ist ein Absperrventil Y6 angeordnet, das auch zu einem Dosierventil weitergebildet sein kann, um einen Teil der Dispersion zum Fermenter und den verbleibenden Teil zurück in den Mischbehälter 4 führen zu können. Von den beiden Rückführmöglichkeiten über die Leitungen 12 und 13 kann in vereinfachten Ausführungen nur eine vorgesehen sein, gegebenenfalls kann eine derartige Rückführmöglichkeit auch gänzlich weggelassen werden.

Mit dem Bezugszeichen 10' ist ein weiterer Feinzerkleinerer angedeutet, der beispielsweise identisch wie der Feinzerkleinerer 10 ausgebildet sein kann. Die Feinzerkleinerer 10 und 10' sind parallel geschaltet. Die über die Leitung 8 aus dem Mischbehälter 4 geführte Suspension wird über eine abzweigende Leitung 8' dem weiteren Feinzerkleinerer 10' zugeführt. Die in den parallel geschalteten Feinzerkleinerern 10 und 10' behandelten Teilströme werden noch stromfaufwärts von dem Homogenisierer 11, im Ausführungsbeispiel auch noch stromaufwärts von der abzweigenden Rückführleitung 12, zusammengeführt. In den parallelen Leitungsästen, in der Leitung 8 und der Leitung 8', ist jeweils ein Absperrventil Y3 und Y4 angeordnet. Die Absperrventile Y3 und Y4 können jeweils zu einem Dosierventil oder einem Mehrwegeventil weitergebildet sein. Der weitere Feinzerkleinerer 10' ist nur in Ausbaustufen der Vorrichtung vorgesehen.

Für den Fall, dass sich im Mischbehälter 4 bereits Biogas in einem nennenswerten Ausmaß bildet, führt eine Gasleitung dieses Biogas aus dem Kopfbereich des Mischbehälters 4 über ein Rückschlagventil Y8 und einen Gasanschluss G in einen Gassammelraum, in dem oder aus dem heraus es beispielsweise mit dem im nachgeschalteten Fermenter erzeugten Biogas gemischt werden kann.

Dem Vorlagebehälter 1 kann ein Erweiterungsbehälter 17 zugeordnet sein, aus dem organische Feststoffe mittels einer Fördereinrichtung 18, 19 in den Vorlagebehälter 1 gefördert werden können.

Die Vorrichtung ist auf einer Plattform P angeordnet. Die Vorgrube 5 und der Erweiterungsbehälter 17 werden nicht zur Vorrichtung gerechnet. Die Plattform P ist in gestrichelter Linie dargestellt. Sie bildet einen Container und wird im Folgenden als "Container-Plattform" bezeichnet. Die gestrichelte Linie umgibt die Komponenten, die in der Container-Plattform P installiert sind. Der Einlass E2 für die Gülle, der Auslass A für die Dispersion und der Auslass G für Gas, ferner ein Auslass für die Sumpfentleerung, sind Anschlüsse an einer oder unterschiedlichen Außenwänden der Container-Plattform P. Optional ist ferner außen an der Container-Plattform ein Hebezeug H angeordnet, beispielsweise eine heb- und senkbare Rampe für ein weiteres Förderzeug, beispielsweise den dargestellten Gabelstapler oder einen Transportbehälter für die organischen Feststoffe, die in den Vorlagebehälter 1 gefüllt werden sollen.

In dem Container P sind unter anderem
- der Vorlagebehälter 1,
- die Zerkleinerer 2, 10 und 11,
- die Fördereinrichtungen 7
- und der Mischbehälter 4
angeordnet. Im Ausführungsbeispiel sind sämtliche Komponenten der beanspruchten Vorrichtung in dem Plattform-Container P angeordnet.

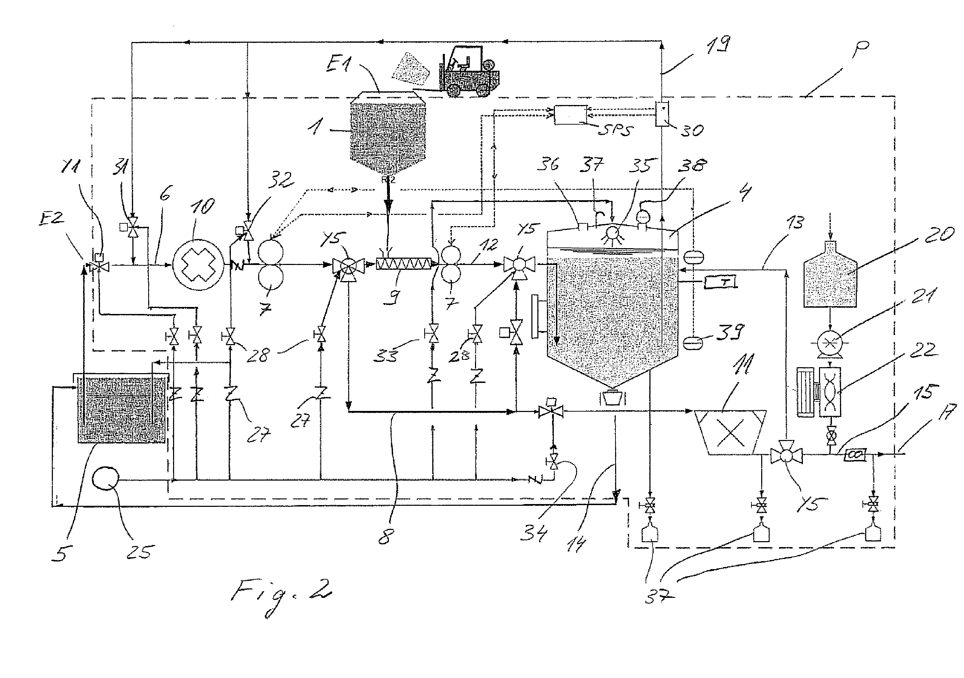

Figur 2 zeigt in einem Verfahrensfließbild eine erfindungsgemäße Vorrichtung eines zweiten Ausführungsbeispiels, deren Komponenten ebenfalls in einer in gestrichelter Linie angedeuteten Container-Plattform P angeordnet sind.

Die Container-Plattform P weist wie im ersten Ausführungsbeispiel Einlässe E1 und E2 für Gülle und organische Feststoffe und einen Auslass A für die Dispersion auf. Der Gasauslass G ist nicht dargestellt. Sie ist über den Auslass A an den Fermenter einer Biogasanlage angeschlossen oder anschließbar. Der Einlass E2 dient als Anschluss an die am Ort der jeweiligen Biogasanlage vorhandene Vorgrube 5. Durch den Einlass E2 kann Gülle, die mit einer oder mehreren anderen Flüssigfraktion(en) vermischt sein kann, beispielsweise mit Gärrückständen, aus der Vorgrube 5 durch die Zuführleitung 6 in den Feinzerkleinerer 10 geführt werden. Diese Fraktion ist über den Zerkleinerer 10 einer Mischeinrichtung 9 zuführbar. Über den anderen Einlass E1 wird der Vorlagebehälter 1 mit den Feststoffen befüllt. Diese Fraktion wird im Vorlagebehälter 1 gelagert und gelangt über eine Austragseinrichtung 2 mit zwei Schnecken in die Mischeinrichtung 9. Die Schnecken der Austragseinrichtung 2 sind in einem konischen unteren Teil des Vorlagebehälters 1 liegend angeordnet. Aus dem Vorlagebehälter 1 können zusätzlich zu der Fraktion aus der nicht in der Container-Plattform P angeordneten Vorgrube 5 weitere Biomassen unterschiedlichster Art zu einer Vorzerkleinerung in die Mischeinrichtung 9 gefüllt werden.

Die Mischeinrichtung 9 kann so ausgebildet sein, dass sie über das Mischen hinaus auch fördert oder zerkleinert. Zusätzlich oder anstelle der Mischeinrichtung 9 kann auch die Austragseinrichtung 2 einen Grobzerkleinerer bilden.

Der Vorlagebehälter 1 ist wie im ersten Ausführungsbeispiel von oben befüllbar, beispielsweise wie dargestellt mittels eines Gabelstaplers oder eines anderen vor Ort verfügbaren Transportmittels. Der nach oben offene Einlass E1 des Vorlagebehälters 1 kann mittels eines Verschlusselements, beispielsweise einer Klappe oder eines Deckels, verschließbar sein, wie auch im ersten Ausführungsbeispiel. Das Verschlusselement kann insbesondere ein Wandungsteil des Containers P sein. Unter der Austragseinrichtung 2 ist ein Auslass des Vorlagebehälters 1 geformt, zweckmäßigerweise einfach als Öffnung. Der Auslass ist lotrecht über einem Einlass der Mischeinrichtung 9 angeordnet, so dass die trockene Biomasse mittels der Austragseinrichtung 2 durch den Auslass des Vorlagebehälters 1 und dann einfach mittels Schwerkraft der Mischeinrichtung 9 zuführbar ist.

Die Zuführleitung 6 führt die Biomasse über den Feinzerkleinerer 10 und die Mischeinrichtung 9 in den Mischbehälter 4. Die Zuführleitung 6 ist mittels eines Steuermittels Y1, im Ausführungsbeispiel ein Absperrventil, das zwischen dem Einlass E2 für die Gülle und dem Zerkleinerer 10 angeordnet ist, absperrbar. In der Zuführleitung 6 sind zwischen dem Zerkleinerer 10 und der Mischeinrichtung 9 eine Pumpe 7 und ein Steuermittel Y5 angeordnet. Zwischen der Mischeinrichtung 9 und dem Mischbehälter 4 sind in der Zuführleitung 6 eine weitere Pumpe 7 und ein weiteres Steuermittel Y5 angeordnet. Die Steuermittel Y5 sind Mehrwegeventile, im Ausführungsbeispiel Dreiwegeventile.

Die Mischeinrichtung 9 vermischt die flüssige oder zumindest fließfähige Biomasse in der Zuführleitung 6 mit der im Wesentlichen aus festen Bestandteilen bestehenden Biomasse aus dem Vorlagebehälter 1. Die Mischeinrichtung 9 ist auch gleichzeitig eine Fördereinrichtung für die Förderung der Biomasse in Richtung auf den Mischbehälter 4. Die Pumpen 7 sind vorzugsweise Drehkolbenpumpen.

In dem Mischbehälter 4 werden die unterschiedlichen Fraktionen auf eine vorgegebene Viskosität eingestellt. Im Behälter 4 kann ein Rührwerk angeordnet sein. Für die Einstellung der Viskosität kann Suspension aus dem Behälter 4 abgezogen und in den Behälter 4 zurückgeführt werden. Falls erforderlich wird die Suspension vor dem Wiedereinleiten über den Feinzerkleinerer 10 oder den Homogenisierer 11 zurück geführt, um die Feststoffanteile nochmals einer Zerkleinerung zu unterziehen oder den zurück geführten Strom oder einen daraus gebildeten Teilstrom der Flüssig-Fest-Mischung in Form einer feinen Dispersion wieder in den Behälter 4 einzuleiten. Desweiteren besteht die Möglichkeit, zur Verringerung der Viskosität über ein Steuermittel 28 Wasser in den Behälter 4 einzuleiten.

Bei dem stromaufwärtigeren Steuermittel Y5 zweigt von der Zuführleitung 6 eine Leitung 8 zu dem Homogenisierer 11 ab. Im Homogenisierer 11 wird diese Biomasse fein zerkleinert, dispergiert und in diesem Sinne homogenisiert. Im Homogenisierer 11 werden die Bestandteile der Biomasse soweit zerkleinert und vermischt, dass eine homogene Flüssigkeit von sahneförmiger Konsistenz entsteht, die als "Maissahne" charakterisiert werden kann. Die Feinzerkleinerung trägt dazu bei, dass die Biomasse im Fermenter rascher und intensiver mikrobiell abgebaut werden kann. Stromab von dem Homogenisierer 11 ist ein weiteres Steuermittel Y5, im Ausführungsbeispiel ein weiteres Mehrwegeventil, angeordnet. Die im Homogenisierer 11 behandelte Biomasse kann über dieses Steuermittel Y5 wahlweise entweder durch eine Rückführleitung 13 in den Mischbehälter 4 oder in einem weiterführenden Teil der Leitung 15 zu dem Auslass A der Vorrichtung geleitet werden. Ferner ist die Aufteilung in zwei Teilströme möglich.

Die Steuermittel Y5 sind so ausgebildet, dass sie den ankommenden Strom unabhängig voneinander jeweils in einem Verhältnis, das von einer Steuerung automatisch vorgegeben und zur Erzeugung einer am Auslass A optimalen Biomasse angepasst wird, in Teilströme aufteilen können. In einer Vereinfachung kann aber auch eines oder können mehrere dieser Steuermittel so ausgebildet sein, dass stets nur ein Ausgang des jeweiligen Steuermittels offen und Durchfluss nur durch diesen Ausgang möglich ist.

Bei dem Homogenisierer 11 handelt es sich wie im ersten Ausführungsbeispiel um ein aus der Kosmetikindustrie bekanntes Dispergiergerät mit einem Elektromotor und einem feinen Schneid- oder Scherwerk, wie es zum Anrühren von Pasten und Salben verwendet wird. Eine Eingangsstufe oder mehrere stromaufwärtige Stufen sind jedoch durch Vergrößerung des Abstands von Scherkanten modifiziert.

Zwecks Umwälzung wird die im Mischbehälter 4 befindliche Mischung aus Flüssigkeit und Festbestandteilen über eine Rückführleitung 19 in den Prozess zurückgeführt. Die Rückführleitung 19 umfasst eine aus dem Mischbehälter 4 führende Steigleitung. Die Rückführleitung 19 verzweigt sich in zwei Leitungszweige, die stromauf von der Mischeinrichtung 9 in die Zuführleitung 6 führen. Der eine Leitungszweig führt stromauf und der andere stromab von dem Zerkleinerer 10 in die Zuführleitung 6. Falls die festen Bestandteile der Mischung aus dem Mischbehälter 4 noch nicht ausreichend fein zerkleinert sind, wird die Mischung über ein absperrbares Steuermittel 31, im Ausführungsbeispiel ein Absperrventil, nochmals durch den Zerkleinerer 10 geführt. Reicht der Zerkleinerungsgrad aus, wird die Mischung unter Umgehung des Zerkleinerers 10 über ein absperrbares Steuermittel 32, im Ausführungsbeispiel ebenfalls ein Absperrventil, zu der Mischeinrichtung 9 geführt, um elektrische Antriebsenergie für den Zerkleinerer 10 einzusparen.

Falls die Biomasse stromab von dem Zerkleinerer 10 bereits fein genug ist, wird sie über das stromauf von der Mischeinrichtung 9 angeordnete Steuermittel Y5 zum Homogenisierer 11 geführt, gegebenenfalls auch nur ein Teilstrom dieser Biomasse, und dort fein dispergiert. Da der Massenstrom der Zuführleitung 6 für den Homogenisierer 11 im Allgemeinen zu groß ist, kann der Strom der Leitung 8 vor dem Homogenisierer 11 geteilt und ein Teilstrom, beispielsweise die Hälfte, zum Homogenisierer 11 und der andere Teilstrom über das Steuermittel Y5 in den Mischbehälter 4 geführt werden.

Über die Einlässe E1 und E2 für die unterschiedlichen Fraktionen der Biomasse hinaus verfügt die Container-Plattform P auch über einen Wasseranschluss 25. Durch den Wasseranschluss 25 kann Wasser zum Verdünnen in den Strom der Biomasse oder zum Spülen von verschmutzten Komponenten eingespeist werden. Falls Komponenten, insbesondere Steuermittel, der Vorrichtung verstopft sein sollten, wird das Steuermittel Y1 zwischen der Vorgrube 5 und dem Zerkleinerer 10 geschlossen und statt der Flüssigkeit aus der Vorgrube 5 die Spülflüssigkeit durch die Anlage, genauer gesagt durch das oder die verstopften Komponenten gepumpt. Hierfür sind die betreffenden Steuermittel über Steuerungsventile 28 und vorzugsweise Rückschlagventile 27 einzeln durchspülbar. Zum Verdünnen kann Wasser über ein Steuermittel 33 und ein vorgeschaltetes Rückschlagventil in den Mischbehälter 4 gefördert werden. Das Wasser wird über Düsen 35 eingespeist, die in dem Mischbehälter 4 angeordnet sind und in Doppelfunktion als Reinigungsdüsen dienen können, um den Mischbehälter 4 auch automatisch reinigen zu können. Wasser kann ferner über ein Steuermittel 34 und ein vorgeschaltetes Rückschlagventil zum Homogenisierer 11 geleitet werden. Die Steuermittel 33 und 34 sind beispielsweise einfache Absperrventile.

Die Container-Plattform P weist auch einen weiteren Auslass-Anschluss auf, über den der Sumpf des Mischbehälters 4 entleert werden kann. Der Mischbehälter 4 ist über eine Leitung 14 und den weiteren Auslass-Anschluss für die Sumpfentleerung an die Vorgrube 5 angeschlossen oder an diese anschließbar.

Die Vorrichtung umfasst eine Steuerung SPS, die über die Steuermittel als Stellglieder, insbesondere die Steuermittel Y5 und 31 bis 34, die Behandlung der Biomasse steuert und regelt. Die Steuerung SPS sorgt dafür, dass die in dem Mischbehälter 4 enthaltene Mischung eine vorgegebene Viskosität zumindest nicht wesentlich unter- oder überschreitet.

Ein Viskosimeter 30 misst die Viskosität der aus dem Mischbehälter 4 zurückgeführten Mischung. Das Viskosimeter 30 ist außerhalb des Mischbehälters 4 in der Rückführleitung 19 stromauf von der Verzweigung der Rückführleitung 19 angeordnet. Solange die Viskosität der Suspension größer ist als ein vorgegebener Viskositätswert, wird dem Mischbehälter 4 über das Steuerungsmittel 33 Wasser zugeführt. Preiswerter und daher bevorzugter wird die Viskosität jedoch indirekt aus der Förderleistung einer oder mehrerer der Fördereinrichtungen der Vorrichtung ermittelt, beispielsweise einer oder mehreren der Pumpen 7 oder einer im Strom der Biomasse näher bei dem Fermenter angeordneten Fördereinrichtung, die vorzugsweise auf dem Plattform-Container P angeordnet ist. Am günstigsten ist es, wenn die Leistung der letzten Fördereinrichtung vor dem Fermenter erfasst wird. Für die indirekte Ermittlung der Viskosität kann insbesondere die Änderung des Stromverbrauchs der betreffenden Fördereinrichtung erfasst werden. Die erfassten Abweichungen von einem Strommittelwert repräsentieren Abweichungen der Viskosität von einem gewünschten Mittelwert, auf den die am Ort der Erfassung vorliegende Mischung, bevorzugt die feine Dispersion am Auslass A, eingestellt werden soll.

Die Ausführungen zur Viskosität, insbesondere zur Ermittlung und Wasserzuführung, und auch zur Wasserspülung von Komponenten gelten für das erste Ausführungsbeispiel gleichermaßen. Desweiteren gilt, dass diejenigen Komponenten beider Ausführungsbeispiele, die mit den gleichen Bezugszeichen versehen sind, nicht nur wenigstens eine gleiche Funktion erfüllen, sondern auch baugleich sein können.

Um Biomasse, die in nennenswertem Umfang tierische Fette oder Rückstände von Antibiotika enthält, behandeln zu können, aber nicht mit den anderen Fraktionen mischen zu müssen, ist in der Container-Plattform P eine Einrichtung zur Behandlung der die tierischen Fette oder Antibiotika enthaltenden Biomasse separat von dem in und aus dem Mischbehälter 4 führenden Leitungssystem angeordnet. Diese zusätzliche Einrichtung umfasst einen Sammelbehälter 20, in dem fetthaltige Abfälle, wie Schlacht- und Küchenabfälle, gesammelt werden. Die Container-Plattform P weist einen weiteren Einlass für diese Fraktion auf. Stromab von dem Sammelbehälter 20 ist ein Zerkleinerer 22 für die Zerkleinerung der aus dem Sammelbehälter 20 zugeleiteten Biomasse angeordnet. Der Zerkleinerer 22 ist ein Ultraschall-Zerkleinerer. Die Zufuhr zu dem Ultraschall-Zerkleinerer 22 erfolgt mittels einer Dosierpumpe 21, die in einer Leitung zwischen dem Sammelbehälter 20 und dem Ultraschall-Zerkleinerer 22 angeordnet ist. Die vom Ultraschall-Zerkleinerer 22 zerkleinerte Biomasse wird stromabwärts von dem Homogenisierer 11 zwischen dem Steuermittel Y5 und dem Auslass A in die Leitung 15 eingespeist.

Die Ausstattung der Container-Plattform P mit der zusätzlichen Einrichtung zur Aufbereitung der tierische Fette oder Antibiotika enthaltenden Biomasse ist in einer Grundvariante der Vorrichtung nicht enthalten. Die Einrichtung ist Bestandteil einer Ausbaustufe der Vorrichtung, findet vorzugsweise aber auch in der Container-Plattform P der Grundversion Platz, so dass die Grundversion problemlos mit dieser Einrichtung nachgerüstet werden kann.

In der Container-Plattform P kann eine Zugabeeinrichtung für Enzyme angeordnet sein, um der Biomasse an geeigneter Stelle, vorzugsweise zwischen dem Homogenisierer 11 und dem Auslass A, in dosierter Menge Enzyme für eine Nachbehandlung beimischen zu können. Eine Beimischung im Mischbehälter 4 ist ebenfalls denkbar, obgleich weniger bevorzugt.

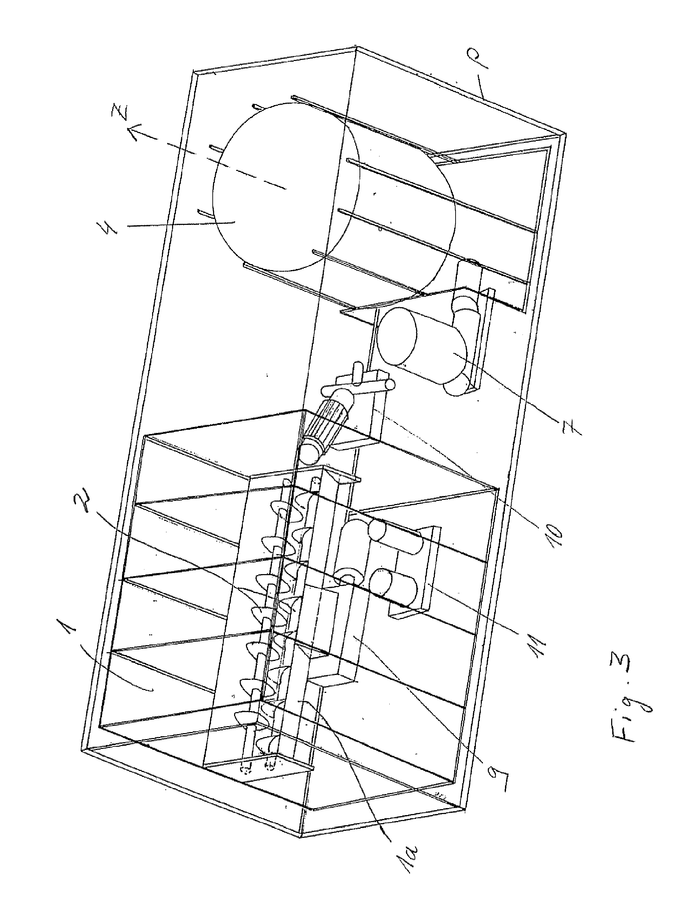

Figur 3 zeigt die Container-Plattform P in einer perspektivischen Sicht. Die Plattform P weist einen Boden, eine Decke und Seitenwände auf. Die Plattform P ist durchsichtig dargestellt, so dass die aufgenommenen Komponenten und deren Anordnung zu erkennen sind. Die Container-Plattform P hat die Form eines länglichen Quaders. Der Quader ist zwischen 2 und 2,5 m breit, zwischen 2 und 4 m hoch und zwischen 5 und 10 m lang. Mit "Z" ist die vertikale Hochachse bezeichnet. Die Container-Plattform P besteht im Wesentlichen aus einem festen Gerippe von Streben, an dem die Außenwände und die Decke befestigt sind, so dass ein allseitig umschlossener Hohlraum erhalten wird, in dem die Komponenten der Vorrichtung angeordnet sind, die die Biomasse aufnehmen, führen, fördern und behandeln.

Der Vorlagebehälter 1 ist im Sinne einer möglichst effizienten Nutzung des verfügbaren Raums in das System aus Gerippe und Containerwänden integriert. indem ein Teil der Außenwände und der Decke gleichzeitig auch einen Teil der Wand und die Decke des Vorlagebehälters 1 bilden. Ferner erstrecken sich Streben des Gerippes schräg zur Hochachse Z geneigt nach innen. Diese Streben tragen einen Boden 1a des Vorlagebehälters 1, so dass sich der Vorlagebehälter 1 in Richtung auf seinen Auslass nach unten verjüngt. Die Wände des Vorlagebehälters 1 sind zumindest innen glatt und weisen einen niedrigen Reibungskoeffizienten auf, um eine Brückenbildung der aufgenommenen Biomasse zu verhindern. Die Wände können beispielsweise an ihrer Innenseite mit einem Gleitmaterial, vorzugsweise einem Kunststoff, beschichtet sein. Zusätzlich oder stattdessen kann eine Rütteleinrichtung vorgesehen sein, mit der die Biomasse im Vorlagebehälter 1 durchgerüttelt werden kann.

Der Mischbehälter 4 ist kein integrierter, sondern ein eingebauter Behälter mit einer runden, um die Hochachse Z zylindrisch umlaufenden Seitenwand.

Die beiden Behälter 1 und 4 sind in Längsrichtung der Container-Plattform P gesehen je nahe bei den Stirnseiten der Container-Plattform P angeordnet, wobei eine der Stirnwände gleichzeitig auch eine Seitenwand des Vorlagebehälters 1 bildet. Die Anordnung der Behälter 1 und 4, die einen großen Teil des Volumens der Container-Plattform P beanspruchen, trägt zur effizienten Nutzung des verfügbaren Raums bei. Für den Zerkleinerer 10, optional die Mischeinrichtung 9, den Homogenisierer 11, die Pumpen 7 und das Leitungssystem mit den Steuermitteln verbleibt genug Raum zwischen den Behältern 1 und 4 und unter dem Vorlagebehälter 1. Der Zerkleinerer 10, die optionale Mischeinrichtung 9, der Homogenisierer 11 und die Pumpen 7 sind leicht zugänglich und so angeordnet und montiert, dass sie einzeln ausgetauscht werden können, ohne den Aus- oder Umbau einer der jeweils anderen Komponenten zu erfordern. Dies erleichtert auch ein nachträgliches Um- oder Aufrüsten der Vorrichtung.

Die Container-Plattform P weist eine Tür auf, durch die man den Innenraum für Reparatur- und Wartungsarbeiten sowie Inspektionen und gegebenenfalls Einstellarbeiten betreten kann. Die Tür befindet sich vorzugsweise an einer Längsseite in einem Bereich zwischen den Behältern 1 und 4.

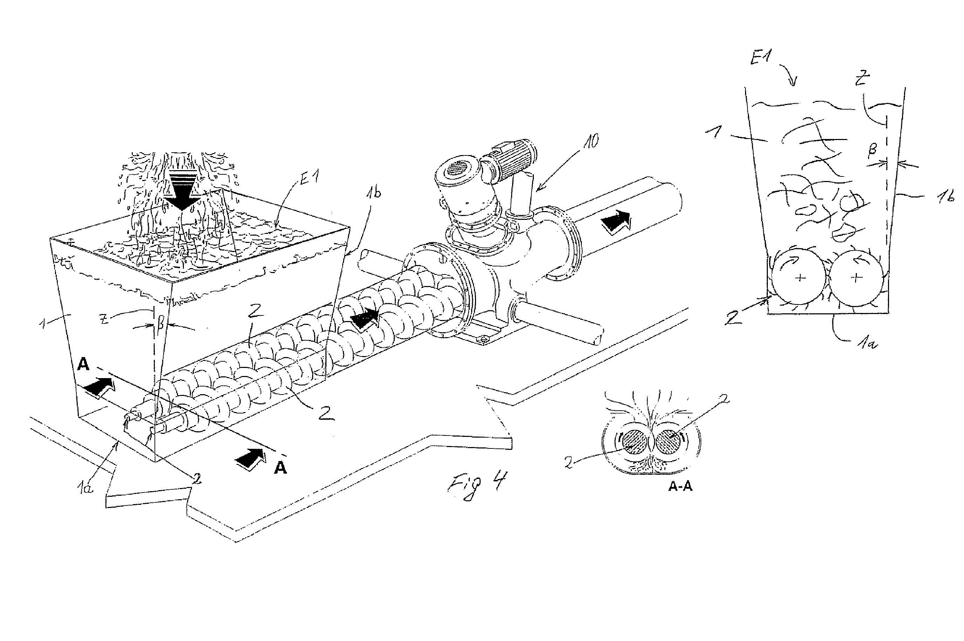

Figur 4 zeigt den Vorlagebehälter 1 in perspektivischer Darstellung, einer Ansicht und einem Schnitt mit dem im unteren Bereich des Behälters 1 angeordneten Grobzerkleinerer 2 des ersten Ausführungsbeispiels. Der Grobzerkleinerer 2 umfasst zwei Förderschnecken, die in der Nähe des Bodens 1a des Behälters 1 angeordnet sind und gegenläufig drehangetrieben werden. Die Förderschnecken sind an ihrem äußeren Umfang mit abragenden Reißzähnen besetzt, wie in der Ansicht angedeutet wird. Die Feststoffe werden von den gegenläufig drehenden Förderschnecken in den Spalt zwischen den Förderschnecken gezogen, unterstützt von der Schwerkraft, dabei gebrochen und zerrissen und entsprechend der Gängigkeit der Förderschnecken axial gefördert. Die Austragseinrichtung 2 des zweiten Ausführungsbeispiels kann durch den Grobzerkleinerer 2 des ersten Ausführungsbeispiels ersetzt werden. In der perspektivischen Darstellung fördern die Förderschnecken die vorzerkleinerten Feststoffe zum Zerkleinerer 10. Dies entspricht einem zu den in den Figuren 1 und 2 dargestellten Verfahrensführungen alternativen Ablauf, in dem der Zerkleinerer 10 im Materialfluss zwischen dem Vorlagebehälter 1 und dem Mischbehälter 4 angeordnet ist.

Einander gegenüberliegende Seitenwände 1b des Vorlagebehälters 1 sind zur Hochachse Z mit einem konstanten Neigungswinkel β geneigt. Die Neigung ist so gewählt, dass sich der Vorlagebehälter 1 nach unten bis auf die in der Draufsicht gesehen von dem Grobzerkleinerer 2 bedeckte Fläche verjüngt, so dass sämtliche Feststoffe vom Grobzerkleinerer 2 erfasst werden können. Die Neigung ist in dem sich verjüngenden Höhenabschnitt des Behälters 1 überall so klein, dass die Feststoffe keine nennenswerten Brücken bilden können und der Grobzerkleinerer sich stets die Feststoffe holen kann. Um einen möglichst kleinen Neigungswinkel β zu erhalten, kann sich der Vorlagebehälter 1 vom Einlass E1 bis auf die Höhe des Grobzerkleinerers 2 überall verjüngen. Der Neigungswinkel β beträgt höchstens 30°, bevorzugter höchstens 20°, und ist im Ausführungsbeispiel kleiner als 20°. Im Sinne eines möglichst kleinen Neigungswinkels β sind beide einander zugewandten Seitenwände 1b relativ zur Hochachse Z geneigt. Der Neigungswinkel β ist im Ausführungsbeispiel konstant, kann in anderen Ausführungsbeispielen jedoch über die Höhe des Vorlagebehälters 1 variieren, vorzugsweise jedoch nur im genannten Winkelbereich.

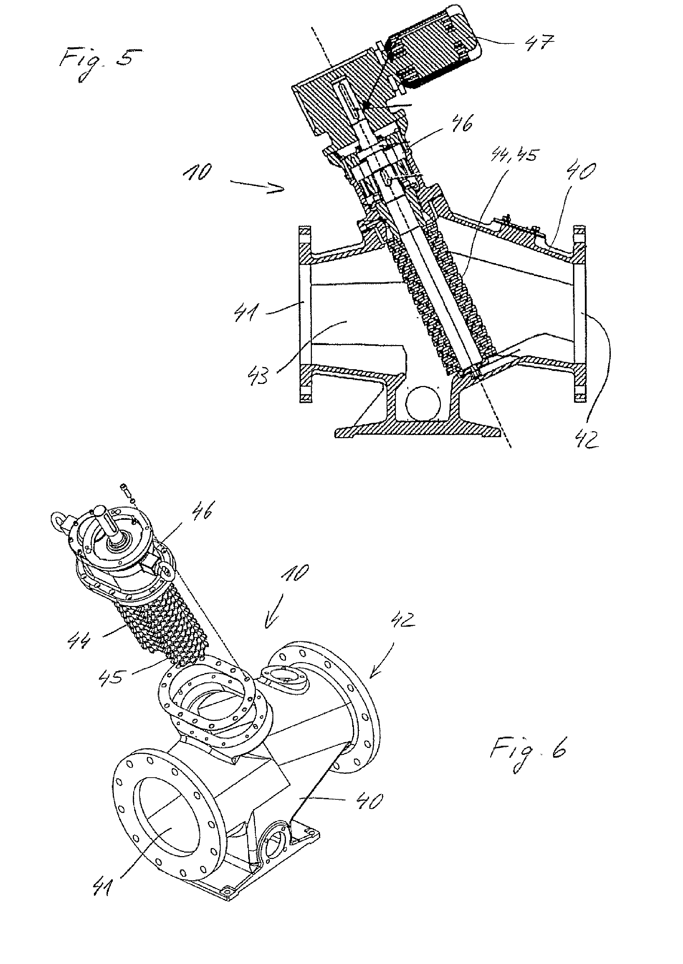

Die Figuren 5 und 6 zeigen den Feinzerkleinerer 10 der beiden Ausführungsbeispiele in einem Schnitt und einer Ansicht. Der Zerkleinerer 10 weist ein Gehäuse 40 mit einem Einlass 41 und einem Auslass 42 auf, zwischen denen das Gehäuse 40 einen Strömungabschnitt 43 der Leitung 8 des ersten oder der Zuführleitung 6 des zweiten Ausführungsbeispiels bildet. Im Strömungsabschnitt 43 sind zwei Messerwellen 44 und 45 quer zu der Strömungsrichtung der Biomasse außenachsig nebeneinander angeordnet. In der Ansicht der Figur 6 ist das ausziehbare Schneidwerk mit den Messerwellen 44 und 45 und einem Getriebe 46 für deren Drehantrieb bereit für die Montage im Gehäuse 40 dargestellt. Jede der Messerwellen 44 und 45 weist axial nebeneinander eine Vielzahl von Schneidmessern mit vorstehenden Schneidzähnen auf. Zwischen nächstbenachbarten Schneidmessern verbleibt jeweils ein axialer Abstand. Die Messerwellen 44 und 45 greifen ineinander, d. h. in die Lücken zwischen den Schneidmessern der einen Messerwelle greifen die Schneidmesser der jeweils anderen ein. Die Messerwellen 44 und 45 werden von einem Motor 47 über das Getriebe 46 gegenläufig drehangetrieben. Zwischen den Messerwellen 44 und 45 verbleibt ein Spalt, durch den die Suspension gefördert und im kämmenden Eingriff der Messerwellen 44 und 45 zerkleinert wird. Die Feststoffbestandteile werden beim Durchgang durch den Spalt zerschnitten, zerrissen und zerdrückt. Die Weite des Spalts, d. h. der Abstand zwischen den Drehachsen der Messerwellen 44 und 45, ist vorzugsweise so gewählt, dass die Feststoffbestandteile nach dem Durchgang durch den Spalt eine größte Erstreckung von höchstens 5 cm haben. Noch bevorzugter ist die Spaltweite so gewählt, dass ihre größte Erstreckung höchstens 2 oder 3 cm beträgt. Für die Zerkleinerung ist es günstig, wenn die Messerwellen 44 und 45 mit unterschiedlicher Umfangsgeschwindigkeit angetrieben werden, zueinander also eine Geschwindigkeitsdifferenz aufweisen.

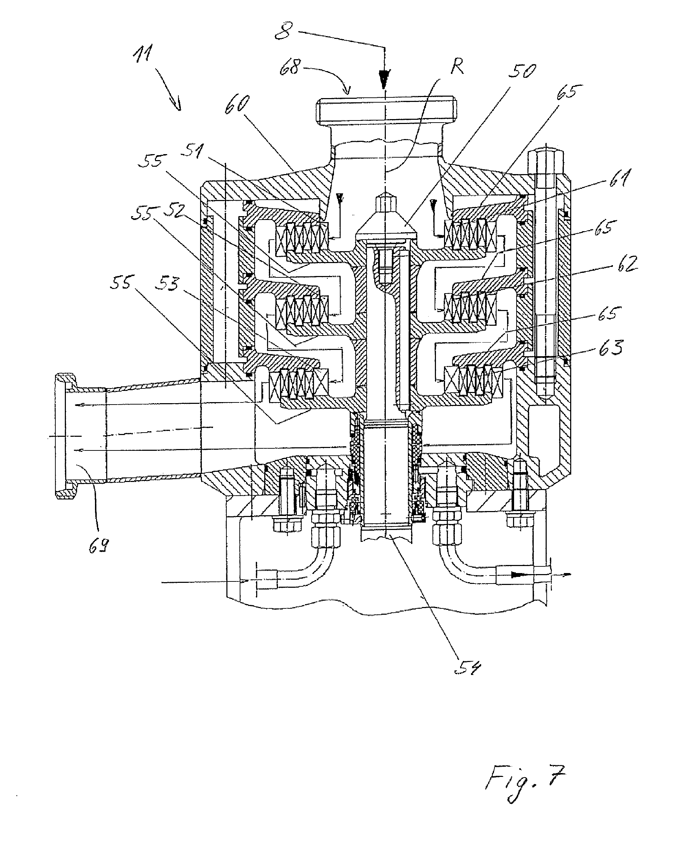

Fig. 7 zeigt den Homogenisierer 11 beider Ausführungsbeispiele in einem Längsschnitt entlang einer zentralen Längsachse, die gleichzeitig eine Rotationsachse R eines Scherwerks des Homogenisierers 11 ist. Der Homogenisierer 11 weist ein Gehäuse 60 mit einem Einlass 68 (Einfüllstutzen) für die zu dispergierende Biomasse und einem Auslass 69 für die dispergierte Biomasse auf. Das Scherwerk besteht aus einem in dem Gehäuse 60 ortsfesten Stator und einem Rotor 50, der in dem Gehäuse 60 um die Rotationsachse R drehbar gelagert ist. Der Stator umfasst eine erste Gruppe von Scherkränzen 61, eine zweite Gruppe von Scherkränzen 62 und eine dritte Gruppe von Scherkränzen 63. Die Scherkränze 61 bis 63 sind unbeweglich mit dem Gehäuse 60 verbunden. Die zur gleichen Gruppe gehörenden Scherkränze, d. h. die Scherkränze 61 der ersten Gruppe, die Scherkränze 62 der zweiten Gruppe und die Scherkränze 63 der dritten Gruppe, umgeben einander je in einem radialen Abstand konzentrisch, während die Scherkränze 61 bis 63 von Gruppe zu Gruppe axial voneinander beabstandet sind. Der Rotor 50 weist eine erste Gruppe von Scherkränzen 51, eine zweite Gruppe von Scherkränzen 52 und eine dritte Gruppe von Scherkränzen 53 auf. Die Scherkränze 51 bis 53 sind mit einer um die Rotationsachse R drehbaren Zentralwelle 54 drehsteif verbunden. Die Scherkränze 51 sind auf einem ersten Träger 55, die Scherkränze 52 auf einem zweiten Träger 55 und die Scherkränze 53 auf einem dritten Träger 55 angeordnet. Die Träger 55 ragen von der Welle 54 nach außen ab und sind axial voneinander beabstandet. Sie bilden je einen Boden für die zu behandelnde Biomasse. Die Scherkränze 51 und 61 der beiden ersten Gruppen umgeben einander konzentrisch, wobei in radialer Richtung auf einen der Scherkränze 51 je einer der Scherkränze 61 folgt, so dass in radialer Richtung eine alternierende Abfolge von Rotor-Scherkränzen 51 und Stator-Scherkränzen 61 erhalten wird. Zwischen den radial benachbarten Scherkränzen 51 und 61 verbleibt jeweils um die Rotationsachse R umlaufend ein schmaler Spalt. Bei den Scherkränzen 52 und 62 der beiden zweiten Gruppen und den Scherkränzen 53 und 63 der beiden dritten Gruppen herrschen die gleichen Verhältnisse.

Jeder der Scherkränze 51 bis 53 weist mehrere Schermesser auf, die je eine Scherkante bilden. Die Scherkränze 61 bis 63 weisen ebenfalls je mehrere Schermesser auf, von denen jedes eine Schergegenkante für die Scherkanten des radial innen oder außen nächsten der Scherkränze 51 bis 53 bildet. Die Scherkanten und Schergegenkanten sind bei jedem der Scherkränze 51 bis 53 und 61 bis 63 um die Rotationsachse R gleichmäßig verteilt angeordnet. Die Scherkanten und Schergegenkanten sind zu der Rotationsachse R parallel. Sie könnten zur Rotationsachse R eine Neigung aufweisen, sollten aber jedenfalls eine zur Rotationsachse R parallele Richtungskomponente haben. Die Scherkanten und Schergegenkanten sind gerade, könnten aber auch einen gekrümmten Verlauf aufweisen.

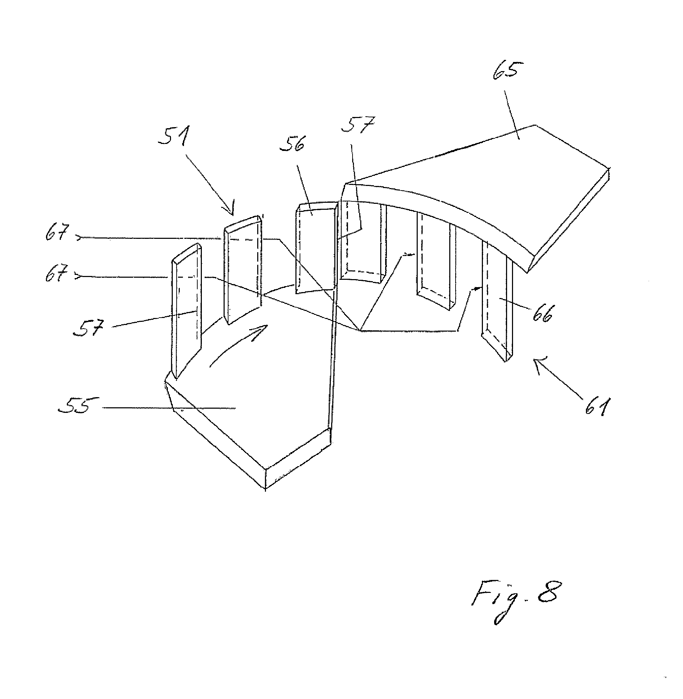

Fig. 8 zeigt einen Bogenabschnitt eines der Scherkränze 51 und einen Bogenabschnitt eines radial nächsten Scherkranzes 61. Der Scherkranz 51 besteht aus Schermessern 56, die von einem Träger 55 des Rotors 50 axial abragen. Der Scherkranz 61 besteht aus Schermessern 66, die von einem der Träger 65 des Gehäuses 60 axial abragen und die Schermesser 56 axial überlappen. Die Träger 55 und 65 sind axial zueinander versetzt. Die Schermesser 56 und 66 ragen von ihrem jeweiligen Träger 55 oder 65 in Richtung auf den jeweils anderen Träger 65 oder 55 zu. An der in Bezug auf die Drehrichtung des Rotors 50 vorlaufenden Seite ist an jedem der Schermesser 56 eine der genannten Scherkanten 57 und an jedem der Schermesser 66 eine der genannten Schergegenkanten 67 geformt. Zwischen den in Umfangsrichtung um die Rotationsachse R benachbarten Schermessern 56 verbleibt jeweils ein lichter Abstand, ebenso zwischen den Schermessern 66, so dass die Biomasse radial durch einen der Scherkränze 51 und 61 in den zwischen nächstbenachbarten Scherkränzen 51 und 61 verbleibenden schmalen Spalt und aus diesem durch den anderen der Scherkränze 51 und 61 strömen kann. Die weiteren Scherkränze 51 bis 53 und 61 bis 63 sind in gleicher Weise geformt und innerhalb der jeweils einander zugeordneten ersten, zweiten und dritten Gruppen so angeordnet, dass innerhalb der einander zugeordneten Gruppen jeweils zwei radial nächste Scherkränze 51 und 61, 52 und 62 sowie 53 und 63 zusammenwirkend eine Zerkleinerungsstufe bilden.

Um die Biomasse zu dispergieren, wird der Rotor 50 durch einen Motor mit großer Geschwindigkeit in Rotation versetzt, vorzugsweise mit einer Umfangsgeschwindigkeit, die am radial äußersten Scherkranz jeder Axialstufe wenigstens 30 m/s beträgt. Zur Kühlung des Scherwerks wird Kühlflüssigkeit durch das System geführt. Die Suspension, optional zusätzlich mit Wasser angereichert, wird am Einlass 68 zugeführt. Sie wird im Gehäuse 60 so geführt, dass sie die Gruppen von Scherkränzen 51 und 61, 52 und 62 sowie 53 und 63 jeweils von radial innen nach außen durchströmt. Ferner ist der Rotor 50 so geformt und angeordnet, dass er der Suspension einen Drehimpuls um die Rotationsachse R verleiht und die erzeugte Fliehkraft die Förderung der Suspension durch den Homogenisierer 11 unterstützt. Gegebenenfalls reicht der Drehantrieb des Rotors 50 für die Förderung der Biomasse im Homogenisierer 11 aus, vorteilhafterweise unterstützt die Fördereinrichtung 7 die Förderung durch den Homogenisierer 11. Die Suspension wird nacheinander durch sämtliche Zerkleinerungsstufen aller Gruppen von Scherkränzen 51 bis 53 und 61 bis 63 gefördert. Der Strömungspfad durch das Gehäuse 60 ist in Figur 7 eingezeichnet. Beim Durchlauf der Suspension durch die Kammern des Scherwerks erfolgt eine sukzessiv feinere Zerkleinerung der festen Bestandteile bis auf eine Partikelgröße von Mikro- und Nanometern. Die Partikel weisen eine größte Erstreckung von vorteilhafterweise höchstens 2 mm, bevorzugter höchstens 1 mm auf.

Die Drehgeschwindigkeit des Rotors 50 wird vorzugsweise so hoch und die radialen Abstände zwischen den miteinander scherenden Schermessern 56 und 66 werden vorzugsweise so klein gewählt, dass in der Flüssigkeit der Biomasse Kavitationsbläschen auftreten, die zu einer feinen Dispergierung der Biomasse beitragen, indem die Kavitationskräfte die festen Bestandteile zerreißen.

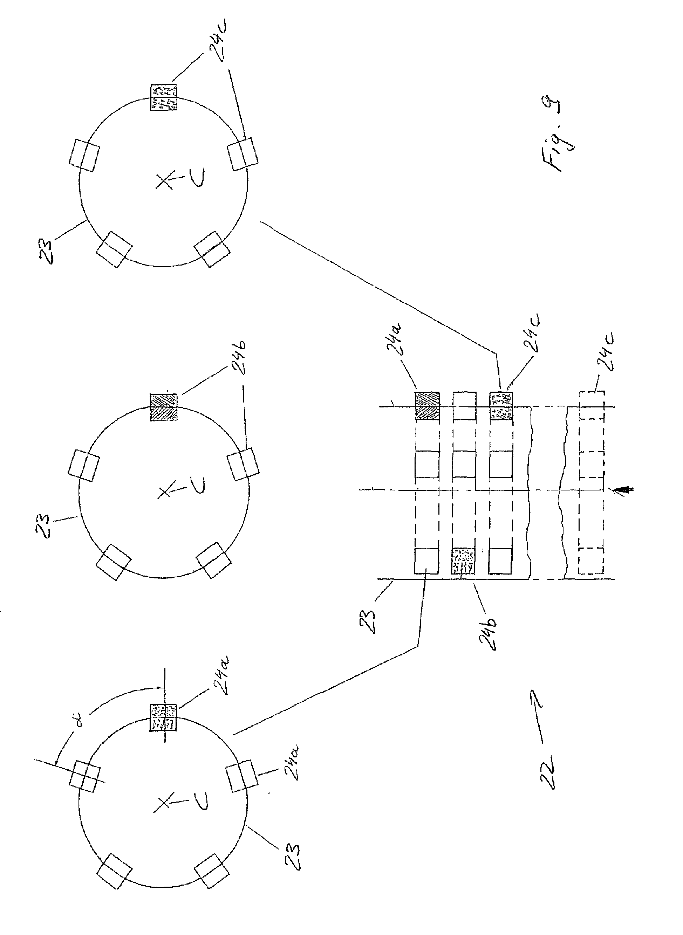

Fig. 9 zeigt den Ultraschall-Zerkleinerer 22, im Ausführungsbeispiel ein Ultraschall-Rohrschwinger, in einem Längsschnitt und drei Querschnitten. Der Ultraschall-Zerkleinerer 22 umfasst ein Rohr 23 und in oder an der Rohrwandung angeordnete Ultraschallerzeuger 24a, 24b und 24c auf. Die Schallerzeuger 24a bilden eine erste Gruppe, die Schallerzeuger 24b eine zweite und die Schallerzeuger 24c eine dritte Gruppe usw. Die Schallerzeuger der jeweils gleichen Gruppe sind in Längsrichtung des Rohrs 23 auf der gleichen Höhe angeordnet, während die Schallerzeuger von Gruppe zu Gruppe in Längsrichtung des Rohrs 23 zueinander versetzt sind. Die Schallerzeuger 24a, 24b und 24c sind innerhalb der jeweiligen Gruppe in einem Winkel α von 72° um ein Zentrum C des jeweiligen Rohrquerschnitts in oder an der Rohrwandung zueinander versetzt positioniert. Da der gesamte Rohrschwinger mit dreißig Schallerzeugern bestückt ist und jede Ebene davon fünf Schallerzeuger aufnimmt, existieren somit sechs Ebenen mit jeweils fünf Schallerzeugern. Die erste Ebene mit den fünf Schwingelementen 24a weist nur einmal ein Schwingelement mit schraffierter Zeichnung aus. Die anderen vier Schwingelemente sind aber auf die gleiche Ansteuerungs-Frequenz abgestimmt. Das gilt für alle nachfolgenden Ebenen, d. h. gleiche Schraffur gleiche Ansteuerungs-Frequenz. Die erste Ebene wird mit einer Frequenz von 20 kHz, die zweite mit einer Frequenz von 100 kHz, und die dritte Ebene wird mit 40 kHz angesteuert. Die nächsten Ebenen - vier, fünf und sechs - sind eine Wiederholung der ersten drei Ebenen. Die sechste Ebene ist nur gestrichelt dargestellt. Die Schwingelemente 24a-c sind von Gruppe zu Gruppe axial fluchtend in oder an der Rohrwandung positioniert. Durch eine Frequenzansteuerung mittels eines Zufallsgenerators der Schwingelemente kann sich für einzelne Partikel der Biomasse keine Fluchtströmung aufbauen, die ein Ausweichen der Partikel zwischen den implodierenden Kavitationsbläschen ermöglichen würde. Da bei der hier vorgeschlagenen Technik keine stehenden Wellen entstehen können, ist ein maximales Zerreißen der Partikel bis in den Nanobereich gegeben.

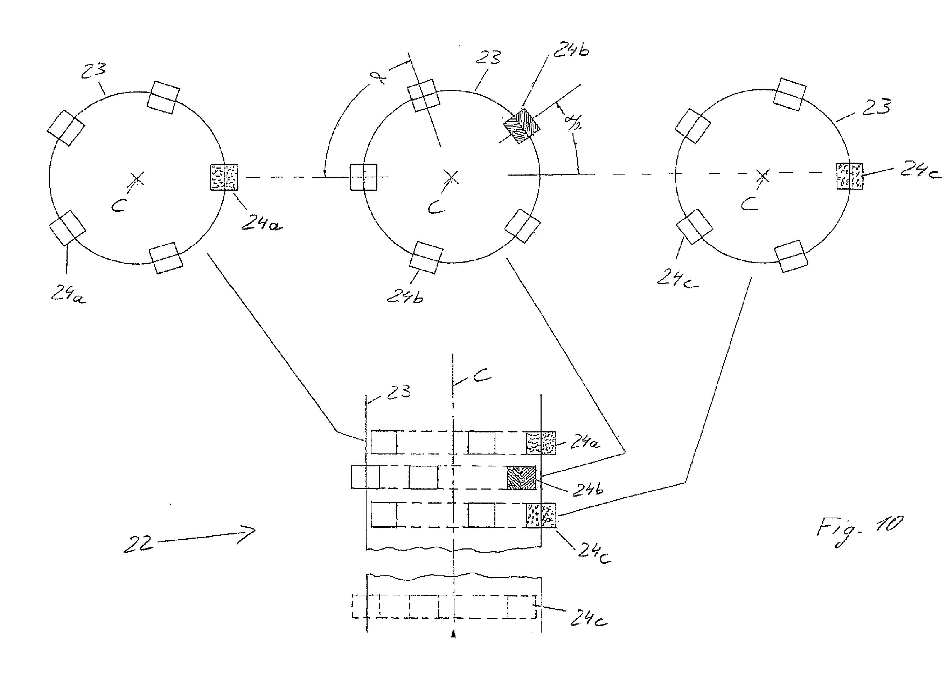

Fig. 10 zeigt eine modifizierte Anordnung von Schallerzeugern 24a-c. Die Schallerzeuger 24b sind hier um α/2, also um 36°, der Drehwinkelposition nach gegenüber den axial links und rechts davon beabstandet angeordneten Schallerzeugern 24a und 24c versetzt. Hier gilt ebenfalls die kontinuierliche Wiederholung der Anordnung der Schallelemente bis zur Ebene sechs.

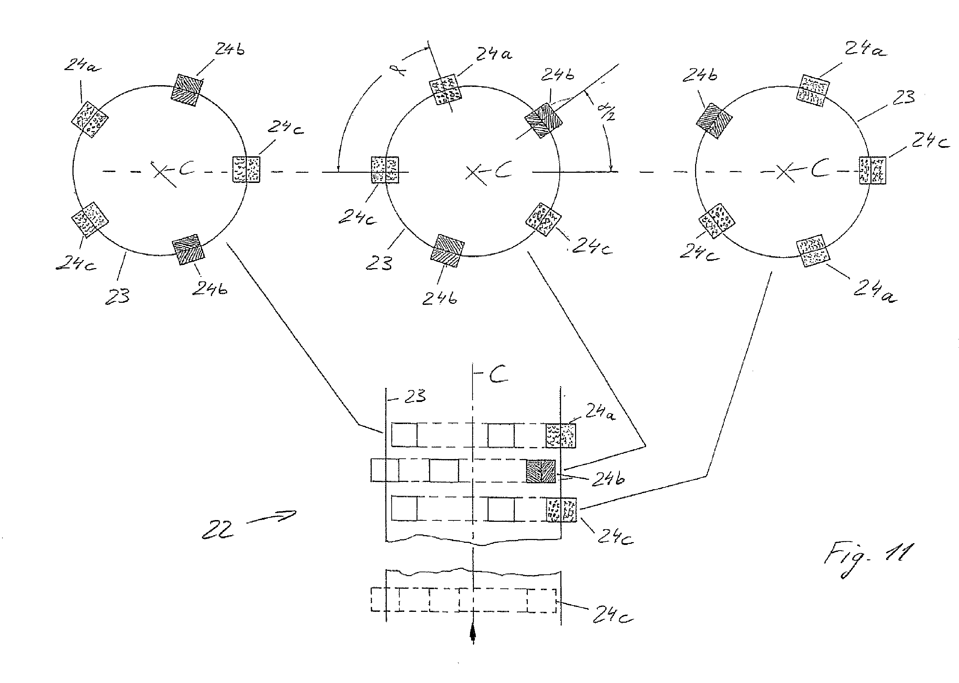

Fig. 11 zeigt noch eine andere Anordnung von Schallerzeugern 24a-c. Die drei der Ansteuerungsfrequenz nach unterschiedlichen Schwingelemente 24a, 24b und 24c sind auf jeder der Ebenen positioniert, wobei sich zwei der drei Schwingelemente auf einer Ebene wiederholen. In Umfangsrichtung um das Zentrum C ergibt sich für die erste Ebene die Abfolge 24a-24b-24c-24b-24c. Die zweite Ebene ist wie bei der Anordnung der Figur 10 um α/2 = 36° verdreht, so dass die Schallerzeuger jeder Ebene zu den Schallerzeugern der axial benachbarten Ebene in Umfangsrichtung jeweils "auf Lücke" positioniert sind. Zusätzlich kann noch die Abfolge verändert werden, wie in dem rechten Querschnitt der Figur 11 angedeutet ist. Diese Veränderung der jeweiligen Position auf einer Ebene setzt sich fort bis zur Ebene sechs.

Die Variationen der Anordnungen der Schallelemente sollen ein Maximum an Kavitationsbläschen erzeugen.

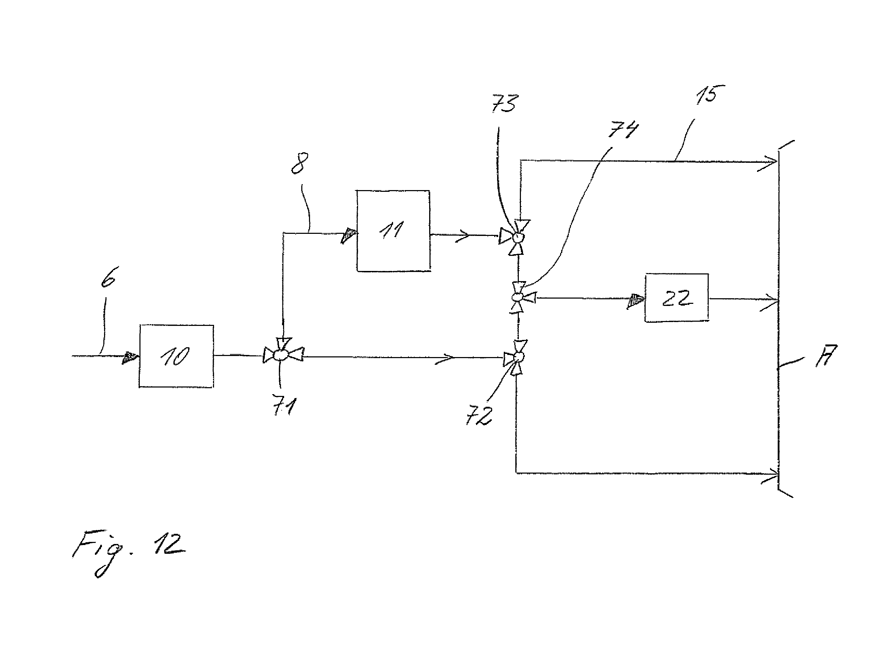

Im Flussdiagramm der Figur 12 sind Variationsmöglichkeiten hinsichtlich der Zerkleinerung von Feststoffbestandteilen dargestellt, wobei der Ultraschall-Zerkleinerer 22 in Abweichung vom Diagramm der Figur 2 in auswählbaren Strömungswegen stromabwärts von entweder dem Feinzerkleinerer 10 oder dem Homogenisierer 11 angeordnet ist. Entsprechend kann eine tierische Fette enthaltende Fraktion über den Feinzerkleinerer 10 und wahlweise den Homogenisierer 11 zum Zerkleinerer 22 geführt werden. Ferner kann die Biomasse nach einer Zerkleinerung mittels des Zerkleinerers 10 unter Umgehung des Homogenisierers 11 zum Auslass A geführt werden.

Die Biomasse wird nach der Zerkleinerung mittels des Feinzerkleinerers 10 über eine erste Verzweigung mit einem ersten Steuermittel 71, beispielhaft ein Dreiwegeventil, zum Homogenisierer 11 oder unter Umgeheung des Homogenisierers 11 zu einer zweiten Verzweigung mit einem zweiten Steuermittel 72, beispielhaft ebenfalls ein Dreiwegeventil, gefördert. Gelangt die Biomasse zum Homogenisierer 11, wird sie nach der weiteren Zerkleinerung und Feindispergierung im Homogenisierer 11 über eine dritte Verzweigung mit einem dritten Steuermittel 73, beispielhaft wieder ein Dreiwegeventil, zum Auslass A oder über ein viertes Steuermittel 74, das beispielhaft ebenfalls ein Dreiwegeventil sein kann, zum Ultraschall-Zerkleinerer 22 gefördert und dort der beschriebenen Ultraschallzerkleinerung unterzogen. Von dort wird die Biomasse zum Auslass A geführt. Bei Umgehung des Homogenisierers 11 wird die Biomasse über die zweite Verzweigung bei 72 entweder unter Umgehung auch des Zerkleinerers 22 oder zwecks Ultraschallbehandlung über die bei 74 gebildete vierte Verzweigung, den Ultraschall-Zerkleinerer 22 und den Auslass A zu dem Fermenter geführt. Der Homogenisierer 11 und der Ultraschall-Zerkleinerer 22 können in einer Variation des Flussdiagramms die Position tauschen.

Die Möglichkeiten der Zuschaltung und Umgehung von wahlweise dem Homogenisierer 11 oder dem Ultraschall-Zerkleinerer 22 einschließlich der Möglichkeit beide Komponenten in Serie dazu zu schalten trägt zur Senkung der Energiekosten bei. Enthält die Biomasse in nennenswertem Ausmaß zellulosehaltige Faserbestandteile, wird der Homogenisierer 11 vorzugsweise zugeschaltet, d. h. die Biomasse wird über den Homogenisierer 11 und über den Ultraschall-Zerkleinerer 22 oder unter Umgehung des Zerkleinerers 22 zum Auslass A gefördert. Enthält die Biomasse in nennenswertem Ausmaß tierische Fette, wird der Zerkleinerer 22 vorzugsweise zugeschaltet, d. h. die Biomasse wird über den Zerkleinerer 22 und über den Homogenisierer 11 oder unter Umgehung des Homogenisierers 11 zum Auslass A gefördert. Fehlen faserige Bestandteile oder tierische Fette, wird oder werden der Homogenisierer 11 oder der Zerkleinerer 22 vorzugsweise umgangen und abgeschaltet.

Das Diagramm der Figur 12 zeigt Möglichkeiten der Modifikation der Vorrichtung beider Ausführungsbeispiele. So wird beispielsweise der Ultraschall-Zerkleinerer 22 in den Strom der Biomasse integriert. Allerdings kann die Vorrichtung in einer weiteren Alternative um die Verzweigungen bei 72, 73 und 74 und die Leitungen ergänzt werden, die vom Homogenisierer 11 zum Zerkleinerer 22 und vom Feinzerkleinerer 10 unter Umgehung des Homogenisierers 11 zum Ultraschall-Zerkleinerer 22 oder gänzlich ohne nachgeschaltete Zerkleinerung vom Zerkleinerer 10 zum Auslass A führen. Das Steuermittel 71 kann das Steuermittel Y5 der Figur 1 oder das stromaufwärtigste Steuermittel Y5 der Figur 2 sein. Das stromabwärts von dem Homogenisierer 11 angeordnete Steuermittel Y5 der Figur 2 oder die Steuermittel Y6 und Y7 der Figur 1 kann oder können beispielsweise zwischen dem Homogenisierer 11 und dem Steuermittel 73 angeordnet sein.

Die Vorrichtung des ersten Ausführungsbeispiels kann wie die des zweiten Ausführungsbeispiels um den Ultraschall-Zerkleinerer 22 ergänzt werden, so dass die Ausführungen zur Zerkleinerung mittels Ultraschall gleichermaßen für das erste Ausführungsbeispiel gelten.

### Bezugszeichen

- 1: Vorlagebehälter
- 1a: Boden
- 1b: Seitenwand
- 2: Grobzerkleinerer, Austragseinrichtung
- 3: Fördereinrichtung
- 4: Mischbehälter
- 5: Vorgrube
- 6: Zuführleitung
- 7: Fördereinrichtung, Pumpe
- 8: Leitung
- 9: Mischeinrichtung
- 10: Feinzerkleinerer
- 11: Homogenisierer
- 12: Rückführleitung
- 13: Rückführleitung
- 14: Rückführleitung
- 15: Auslassleitung
- 16: Gasabführung
- 17: Erweiterungsbehälter
- 18: Fördereinrichtung
- 19: Rückführleitung
- 20: Sammelbehälter
- 21: Dosierpumpe
- 22: Ultraschall-Zerkleinerer
- 23: Rohr
- 24: Ultraschallerzeuger
- 25: Wasseranschluss
- 26: Steuermittel, Absperrventil
- 27: Rückschlagventil
- 28: Steuermittel, Absperrventil
- 29: -
- 30: Viskosimeter
- 31: Steuermittel, Absperrventil
- 32: Steuermittel, Absperrventil
- 33: Steuermittel, Absperrventil
- 34: Steuermittel, Absperrventil
- 35: Düse
- 36: Schauglas
- 37: Entlüftung
- 38: Aufsetzbare Lampe
- 39: Füllstandssensor
- 40: Gehäuse
- 41: Einlass
- 42: Auslass
- 43: Strömungsabschnitt
- 44: Messerwelle
- 45: Messerwelle
- 46: Getriebe
- 47: Motor
- 48: -
- 49: -
- 50: Rotor
- 51: Scherkranz
- 52: Scherkranz
- 53: Scherkranz
- 54: Welle
- 55: Träger
- 56: Schermesser
- 57: Scherkante
- 58: -
- 59: -
- 60: Gehäuse
- 61: Scherkranz
- 62: Scherkranz
- 63: Scherkranz
- 64: -
- 65: Träger
- 66: Schermesser
- 67: Schergegenkante
- 68: Einlass
- 69: Auslass
- 70: -
- 71: Steuermittel, Mehrwege-Dosierventil oder -Absperrventil
- 72: Steuermittel, Mehrwege-Dosierventil oder -Absperrventil
- 73: Steuermittel, Mehrwege-Dosierventil oder -Absperrventil
- 74: Steuermittel, Mehrwege-Dosierventil oder -Absperrventil

- A: Auslass für die Dispersion
- C: Zentrum des Rohrquerschnitts
- E1: Einlass
- E2: Einlass
- G: Auslass für Gas
- H: Hebezeug
- P: Plattform, Container-Plattform
- R: Rotationsachse
- SPS: Steuerung
- Z: Hochachse
- Y0: Steuermittel, Absperrventil
- Y1: Steuermittel, Absperrventil
- Y2: Steuermittel, Dosier- oder Absperrventil
- Y3: Steuermittel, Dosier- oder Absperrventil
- Y4: Steuermittel, Dosier- oder Absperrventil
- Y5: Steuermittel, Mehrwege-Dosierventil oder -Absperrventil
- Y6: Steuermittel, Dosier- oder Absperrventil
- Y7: Steuermittel, Dosier- oder Absperrventil
- Y8: Steuermittel, Rückschlagventil

- α: Winkelabstand
- β: Neigungswinkel

## Patentansprüche

1. Verfahren zur Aufbereitung von Gülle und organischen Feststoffen für eine Fermentierung, bei dem
a) die organischen Feststoffe mittels einer ersten Zerkleinerungseinrichtung (2; 2, 10; 2, 9; 2, 9, 10) zerkleinert,
b) vor oder nach der Zerkleinerung in der Gülle suspendiert,
c) nach der Zerkleinerung in Suspension mittels einer zweiten Zerkleinerungseinrichtung (10, 11; 11) auf eine größte Erstreckung von maximal 5 mm feiner zerkleinert
d) und in der Suspension gleichmäßig dispergiert werden
e) und die Dispersion direkt oder nach weiterer Behandlung der Fermentierung zugeführt wird.

2. Verfahren nach Anspruch 1, das wenigstens eines der folgenden Merkmale aufweist:
- die erste Zerkleinerungseinrichtung (2; 2, 10; 2, 9; 2, 9, 10) bricht voluminöse und zerreißt faserige Feststoffe, vorzugsweise in einem Trockengemisch,
- wenigstens eine der Zerkleinerungseinrichtungen zerschneidet und zerreißt und vorzugsweise auch zerdrückt die Feststoffe, vorzugsweise in Suspension,
- die zweite Zerkleinerungseinrichtung (11) zerkleinert die Feststoffe in Suspension mittels wenigstens einem aus Scheren und Kavitation.

3. Verfahren nach einem der vorhergehenden Ansprüche, bei dem ein Grobzerkleinerer (2; 9) der ersten Zerkleinerungseinrichtung (2; 2, 10; 2, 9; 2, 9, 10) die Feststoffe auf eine größte Erstreckung von maximal 30 cm, vorzugsweise maximal 20 cm, und ein stromabwärts von dem Grobzerkleinerer (2; 9) angeordneter Feinzerkleinerer (10; 11) der ersten Zerkleinerungseinrichtung oder der zweiten Zerkleinerungseinrichtung die grobzerkleinerten Feststoffe auf die größte Erstreckung von maximal 5 mm zerkleinern.

4. Verfahren nach einem der vorhergehenden Ansprüche, bei dem die Feststoffe mittels der ersten Zerkleinerungseinrichtung (2, 10; 2, 9, 10) auf eine größte Erstreckung von maximal 5 cm zerkleinert werden.

5. Verfahren nach einem der vorhergehenden Ansprüche, bei dem die Feststoffe der Suspension mittels der zweiten Zerkleinerungseinrichtung (10, 11; 11) auf eine größte Erstreckung von maximal 2 mm, vorzugsweise maximal 1 mm, zerkleinert und vorzugsweise bei der Zerkleinerung gleichmäßig dispergiert werden.

6. Verfahren nach einem der vorhergehenden Ansprüche, bei dem die Feststoffe in einem Feststoffgemisch mittels eines Grobzerkleinerers (2; 9) der ersten Zerkleinerungseinrichtung (2; 2, 10; 2, 9; 2, 9, 10) zerkleinert, nach der Zerkleinerung in der Gülle suspendiert und in Suspension mittels eines stromabwärts von dem Grobzerkleinerer (2; 9) angeordneten Feinzerkleinerers (10; 11) der ersten Zerkleinerungseinrichtung (2, 10; 2, 9, 10) oder der zweiten Zerkleinerungseinrichtung (10, 11; 11) feiner zerkleinert werden.

7. Verfahren nach einem der vorhergehenden Ansprüche, bei dem
- die Feststoffe mittels eines Grobzerkleinerers (2; 9) der ersten Zerkleinerungseinrichtung,
- mittels eines stromabwärts von dem Grobzerkleinerer (2; 9) angeordneten Feinzerkleinerers (10) und
- mittels eines stromabwärts von dem Feinzerkleinerer (10) angeordneten Homogenisierers (11) der zweiten Zerkleinerungseinrichtung (11) zerkleinert
- und dem Homogenisierer (11), vorzugsweise bereits dem Feinzerkleinerer (10), in Suspension mit der Gülle zugeführt werden.

8. Vorrichtung für die Aufbereitung von Gülle und organischen Feststoffen für eine Fermentierung, die Vorrichtung umfassend:
a) eine Zuführung (E1, 1) für die Feststoffe,
b) eine Zuführung (E2, 6) für die Gülle,
c) eine Mischeinrichtung (4; 9) zum Mischen der Feststoffe und der Gülle,
d) eine erste Zerkleinerungseinrichtung (2; 2, 10; 2, 9; 2, 9, 10) für die in einem Trockengemisch oder in Suspension mit der Gülle vorliegenden Feststoffe
e) und eine stromabwärts von der ersten Zerkleinerungseinrichtung (2; 2, 10; 2, 9; 2, 9, 10) und der Mischeinrichtung (4; 9) angeordnete zweite Zerkleinerungseinrichtung (10, 11; 11) für die in Suspension mit der Gülle vorliegenden Feststoffe,
f) wobei die zweite Zerkleinerungseinrichtung (10, 11; 11) für eine feinere Zerkleinerung der mittels der ersten Zerkleinerungseinrichtung (2; 2, 10; 2, 9; 2, 9, 10) vorzerkleinerten Feststoffe ausgebildet ist.

9. Vorrichtung nach dem vorhergehenden Anspruch, **dadurch gekennzeichnet, dass** die Zuführung (E1, 1) für die Feststoffe einen Behälter (1) für die Feststoffe, vorzugsweise für ein Trockengemisch der Feststoffe, umfasst und wenigstens eines der folgenden Merkmale erfüllt ist:
- in einem unteren Bereich oder unter einem Auslass des Behälters (1) ist ein Grobzerkleinerer (2; 9) der ersten Zerkleinerungseinrichtung (2; 2, 10; 2, 9; 2, 9, 10) angeordnet,
- in einem unteren Bereich oder unter einem Auslass des Behälters (1) ist ein Förderer (2) für die Feststoffe angeordnet und der Behälter (1) verjüngt sich trichterförmig in Richtung auf den Förderer (2),
- in einem unteren Bereich oder unter einem Auslass des Behälters (1) ist eine Förderschnecke (2) für die Feststoffe mit horizontaler Drehachse angeordnet,
- der Behälter (1) weist eine Öffnung (E1) auf, durch die er von oben mit den Feststoffen befüllbar ist.

10. Vorrichtung nach einem der vorhergehenden Ansprüche, umfassend einen Flüssigkeitsbehälter (4) für die Suspension, wobei wenigstens eines der folgenden Merkmale erfüllt ist:
- Gülle ist über die Zuführung (E2, 6) direkt in den Flüssigkeitsbehälter (4) führbar,
- der Flüssigkeitsbehälter (4) bildet die Mischeinrichtung,
- der Flüssigkeitsbehälter (4) ist stromabwärts von einem Grobzerkleinerer (2; 9) der ersten Zerkleinerungseinrichtung (2; 2, 10; 2, 9; 2, 9, 10) angeordnet,
- der Flüssigkeitsbehälter (4) ist stromaufwärts von der zweiten Zerkleinerungseinrichtung (10, 11; 11) angeordnet,
- stromabwärts von einem Feinzerkleinerer (10; 11) der ersten Zerkleinerungseinrichtung oder der zweiten Zerkleinerungseinrichtung ist zumindest ein Teilstrom der Suspension mit den fein zerkleinerten Feststoffpartikeln über eine Rückführleitung (12; 13), die vorzugsweise von einer weiterführenden Leitung (15) abzweigt, in den Flüssigkeitsbehälter (4) führbar.

11. Vorrichtung nach einem der vorhergehenden Ansprüche, die wenigstens eines der folgenden Merkmale aufweist:
- die erste Zerkleinerungseinrichtung (2; 2, 10) umfasst ein Brech- und Reißwerk (2) zum Brechen voluminöser Feststoffe, beispielsweise Kartoffeln oder Rüben, und Zerreißen fasriger Feststoffe, beispielsweise Gras, Heu oder Stroh oder von Ballen fasriger Feststoffe,
- wenigstens eine der Zerkleinerungseinrichtungen umfasst ein Schneid- und Reißwerk (10) zum Zerschneiden, Zerreißen und vorzugsweise Zerdrücken der Feststoffe,
- die zweite Zerkleinerungseinrichtung (11) umfasst ein Scher- und Dispergierwerk (11).

12. Vorrichtung nach dem vorhergehenden Anspruch, **dadurch gekennzeichnet, dass** das Brech- und Reißwerkzeug (2) wenigstens eines der folgenden Merkmale erfüllt:
- es weist eine Förderschnecke mit abragenden Reißzähnen auf,
- es weist einen Rotor mit abragenden Reißzähnen auf, der in einem unteren Bereich eines Behälters (1) für die Feststoffe angeordnet ist und mit wenigstens einer reißzahnbewährten Wand des Behälters oder einem zweiten Rotor mit abragenden Reißzähnen das Brech- und Reißwerk (2) bildet,
- es weist einen ersten Rotor mit abragenden Reißzähnen und einen zweiten Rotor mit abragenden Reißzähnen auf, wobei die Rotoren gegenläufig drehantreibbar und nebeneinander so angeordnet sind, dass ihre Reißzähne bei einem Drehantrieb miteinander kämmen.

13. Vorrichtung nach einem der vorhergehenden Ansprüche, **dadurch gekennzeichnet, dass** eine der Zerkleinerungseinrichtungen einen Feinzerkleinerer (10) zum Zerschneiden, Zerreißen und vorzugsweise Zerdrücken der Feststoffe umfasst und wenigstens eines der folgenden Merkmale erfüllt:
- der Feinzerkleinerer (10) weist drehantreibbare Messerwellen (44, 45) auf mit jeweils mehreren Gruppen von um die jeweilige Wellenachse umlaufend angeordneten Schneidzähnen, wobei die Gruppen jeder der Messerwellen (44, 45) axial voneinander beabstandet sind und die Messerwellen (44, 45) ineinander greifen, so dass durch einen Spalt zwischen den Messerwellen geförderte Feststoffe durch wenigstens eines aus Schneiden, Reißen und Drücken zerkleinert werden, und wobei die Messerwellen zueinander vorzugsweise außenachsig angeordnet sind,
- der Feinzerkleinerer (10) weist drehantreibbare Messerwellen (44, 45) auf, die nebeneinander angeordnet sind, so dass zwischen den Messerwellen ein von der Suspension durchströmbarer, quer zur Strömungsrichtung der Suspension erstreckter Spalt verbleibt,
- die eine der Zerkleinerungseinrichtungen umfasst einen weiteren solchen Feinzerkleinerer (10') und die Feinzerkleinerer (10, 10') sind im Strom der Suspension parallel geschaltet, um den Durchsatz zu erhöhen.

14. Vorrichtung nach einem der vorhergehenden Ansprüche, **dadurch gekennzeichnet, dass** die zweite Zerkleinerungseinrichtung (10, 11; 11) einen Homogenisierer (11) umfasst und wenigstens eines der folgenden Merkmale erfüllt:
- der Homogenisierer (11) weist ein mehrstufiges Scherwerk mit ersten Scherkränzen (51, 52, 53) und zweiten Scherkränzen (61, 62, 63) auf, wobei die ersten Scherkränze relativ zu den zweiten Scherkränzen um eine Drehachse (R) drehbar sind und jeweils einer der ersten Scherkränze einen der zweiten Scherkränze umgibt oder von einem der zweiten Scherkränze umgeben wird,
- der Homogenisierer (11) ist in einem durch einen Mischbehälter (4) und den Homogenisierer (11) führbaren Strom der Suspension näher bei einem Auslass (A) der Vorrichtung angeordnet als der Mischbehälter (4),
- die Dispersion mit den mittels des Homogenisierers (11) fein zerkleinerten Feststoffen ist in einen Mischbehälter (4) führbar,
- die Dispersion mit den mittels des Homogenisierers (11) fein zerkleinerten Feststoffen ist wahlweise zu einem Auslass (A) der Vorrichtung oder in einen Mischbehälter (4) führbar.

15. Vorrichtung nach einem der vorhergehenden Ansprüche, **dadurch gekennzeichnet, dass**
- die zweite Zerkleinerungseinrichtung (10, 11; 11) einen Homogenisierer (11) umfasst, der einen ersten Scherkranz (51) und zweiten Scherkranz (61) aufweist, wobei der erste Scherkranz (51) relativ zu dem zweiten Scherkranz (62) um eine Rotationsachse (R) drehantreibbar ist und wobei vorzugsweise einer der Scherkränze den anderen umgibt,
- der erste Scherkranz (51) um die Rotationsachse (R) angeordnete, quer zu einer Umfangsrichtung des ersten Scherkranzes (51) weisende Scherkanten (57) und der zweite Scherkranz (61) um die Rotationsachse (R) angeordnete, quer zu der Umfangsrichtung weisende Schergegenkanten (67) aufweist
- und die Suspension in einen zwischen den Scherkränzen (51, 61) um die Rotationsachse (R) verlaufenden Spalt förderbar ist, so dass sie bei einem Drehantrieb des ersten Scherkranzes (51) mittels der miteinander scherenden Scher- und Schergegenkanten (57, 67) zerkleinert wird.

16. Vorrichtung nach dem vorhergehenden Anspruch, **dadurch gekennzeichnet, dass** der Homogenisierer (11) einen vorzugsweise drehantreibbaren dritten Scherkranz (51) mit um die Rotationsachse (R) angeordneten, quer zu einer Umfangsrichtung des dritten Scherkranzes weisenden Scherkanten (57) oder Schergegenkanten umfasst und die Suspension aus dem Spalt in einen zwischen dem zweiten und dem dritten Scherkranz um die Rotationsachse (R) verlaufenden weiteren Spalt förderbar ist, so dass sie bei einem Drehantrieb des ersten Scherkranzes (51) auch mittels der miteinander scherenden Scher- und Schergegenkanten (57, 67) des zweiten und des dritten Scherkranzes zerkleinert wird.

17. Vorrichtung nach dem vorhergehenden Anspruch, **dadurch gekennzeichnet, dass** der Homogenisierer (11) einen vierten Scherkranz (61) mit um die Rotationsachse (R) angeordneten, quer zu der Umfangsrichtung weisenden Schergegenkanten (67) umfasst, wobei der dritte und der vierte Scherkranz vorzugsweise einander umgeben, und dass die Suspension aus dem zwischen dem ersten und dem zweiten Scherkranz gebildeten Spalt in einen zwischen dem dritten und dem vierten Scherkranz um die Rotationsachse (R) verlaufenden weiteren Spalt förderbar ist, so dass sie bei einem Drehantrieb des ersten und vorzugsweise des dritten Scherkranzes (51) auch mittels der miteinander scherenden Scher- und Schergegenkanten (57, 67) des dritten und des vierten Scherkranzes zerkleinert wird.

18. Vorrichtung nach dem vorhergehenden Anspruch, **dadurch gekennzeichnet, dass** der dritte und der vierte Scherkranz den ersten und den zweiten Scherkranz umgeben oder von dem ersten und dem zweiten Scherkranz umgeben werden, so dass die Scherkränze radial hintereinander drei jeweils um die Rotationsachse (R) verlaufende Spalte bilden, und dass die Biomasse sukzessive in radialer Richtung durch diese Spalte förderbar ist, so dass sie bei einem Drehantrieb des ersten und vorzugsweise des dritten Scherkranzes (51) in jedem der Spalte mittels der miteinander scherenden Scher- und Schergegenkanten (57, 67) der radial jeweils nächstbenachbarten Scherkränze zerkleinert wird.

19. Vorrichtung nach Anspruch 17, **dadurch gekennzeichnet, dass** der erste und der zweite Scherkranz (51, 61) axial vor dem dritten und dem vierten Scherkranz (52, 62) angeordnet sind und die Biomasse sukzessive durch die Spalte förderbar ist, so dass sie bei einem Drehantrieb des ersten und des dritten Scherkranzes (51, 52) in den Spalten mittels der jeweils miteinander scherenden Scherkanten (57) und Schergegenkanten (67) zerkleinert wird.

20. Vorrichtung nach einem der fünf vorhergehenden Ansprüche, **dadurch gekennzeichnet, dass** wenigstens einer der Scherkränze zwischen in Umfangsrichtung benachbarten Scherkanten oder Schergegenkanten radial durchlässig ist, um die Biomasse in radialer Richtung in den mittels des wenigstens einen der Scherkränze gebildeten Spalt zu fördern.

21. Vorrichtung nach einem der vorhergehenden Ansprüche, **dadurch gekennzeichnet, dass** die erste Zerkleinerungeinrichtung (2; 2, 10; 2, 9; 2, 9, 10) einen Grobzerkleinerer (2; 9) für eine grobe und eine der Zerkleinerungseinrichtungen einen stromabwärts von dem Grobzerkleinerer (2; 9) angeordneten Feinzerkleinerer (10) für eine feinere Zerkleinerung der Feststoffe aufweist.

22. Vorrichtung nach dem vorhergehenden Anspruch, **dadurch gekennzeichnet, dass** die zweite Zerkleinerungeinrichtung (10, 11; 11) einen stromabwärts von dem Feinzerkleinerer (10) angeordneten Homogenisierer (11) für eine nochmals feinere Zerkleinerung der Feststoffe aufweist.

23. Vorrichtung nach einem der zwei vorhergehenden Ansprüche, **dadurch gekennzeichnet, dass** zur Vergrößerung des Durchsatzes wenigstens zwei Feinzerkleinerer (10, 10') oder Homogenisierer (11) in Parallelschaltung stromabwärts von dem Grobzerkleinerer (2; 9) angeordnet sind.

24. Vorrichtung nach einem der drei vorhergehenden Ansprüche, **gekennzeichnet durch** wenigstens eines der folgenden Merkmale:
- der Grobzerkleinerer (2; 9) ist für eine Zerkleinerung der Feststoffe auf eine größte Erstreckung von maximal 30 cm, vorzugsweise maximal 20 cm, ausgebildet,
- der Feinzerkleinerer (10) ist für eine Zerkleinerung der Feststoffe auf eine größte Erstreckung von maximal 5 cm, vorzugsweise maximal 2 cm, ausgebildet,
- der Homogenisierer (11) ist für eine Zerkleinerung der Feststoffe auf eine größte Erstreckung von maximal 2 mm, vorzugsweise maximal 1 mm, ausgebildet.

25. Vorrichtung nach einem der vorhergehenden Ansprüche, umfassend einen Zerkleinerer (22), vorzugsweise Ultraschall-Zerkleinerer, für tierische Fette enthaltende Biomasse, wobei die tierische Fette enthaltende Biomasse über diesen Zerkleinerer (22) vorzugsweise unter Umgehung eines Mischbehälters (4) zu dem Auslass (A) führbar ist.

26. Vorrichtung nach einem der vorhergehenden Ansprüche, **dadurch gekennzeichnet, dass** ein Einlass (E1, E2) für die Gülle oder die organischen Feststoffe oder eine Teilfraktion der organischen Feststoffe, ein Auslass (A) für die mittels wenigstens einer der Zerkleinerungseinrichtungen behandelte Suspension und wenigstens zwei der folgenden Komponenten
- ein Zerkleinerer der ersten Zerkleinerungseinrichtung (2; 2, 10; 2, 9; 2, 9, 10),
- ein Zerkleinerer der zweiten Zerkleinerungseinrichtung (10, 11; 11),
- ein Ultraschall-Zerkleinerer (22), der als zusätzlicher Zerkleinerer zu der ersten oder zweiten Zerkleinerungseinrichtung gehören kann,
- die Mischeinrichtung (4; 9),
- eine Pumpe (7) zum Fördern der Suspension,
- ein Vorlagebehälter (1) zur Aufnahme der organischen Feststoffe oder einer Teilfraktion der organischen Feststoffe
auf einer gemeinsamen Plattform (P) angeordnet sind, die mit den darauf angeordneten Komponenten als Einheit auf der Straße oder Schiene transportabel ist.

27. Vorrichtung nach dem vorhergehenden Anspruch, **dadurch gekennzeichnet, dass** die Plattform (P) mit den darauf angeordneten Komponenten über alles eine Breite (B) von höchstens 3 m oder Höhe (H) von höchstens 5 m aufweist.

28. Vorrichtung nach einem der zwei vorhergehenden Ansprüche, **dadurch gekennzeichnet, dass** die Plattform (P) einen Container bildet, der vorzugsweise begehbar ist, und dass wenigstens eine der Komponenten in dem Container angeordnet ist.

29. Vorrichtung nach einem der vorhergehenden Ansprüche, **dadurch gekennzeichnet, dass** ein Wasseranschluss (25) für die Einspeisung von Wasser, vorzugsweise in einen Mischbehälter (4) oder Homogenisierer (11), oder für eine Spülung von Komponenten (Yi, 26, 31-34), vorzugsweise Steuermittel, der Vorrichtung angeordnet ist, vorzugsweise auf der Plattform (P) nach Anspruch 26.

30. Vorrichtung nach einem der vorhergehenden Ansprüche, **dadurch gekennzeichnet, dass** sie eine Einrichtung (7, 30) zur Ermittlung der Viskosität eines Stroms von mittels der Vorrichtung behandelter Biomasse und eine Steuerung umfasst, die in Abhängigkeit von der ermittelten Viskosität eine Fördereinrichtung (7) oder ein Steuermittel (Yi, 26, 31-34), vorzugsweise ein Dosier- oder Absperrventil oder Mehrwegeventil, ansteuert, über das der Suspension oder Dispersion Flüssigkeit beimischbar ist, wobei die Einrichtung (7, 30) zur Ermittlung der Viskosität oder die Steuerung (SPS) vorzugsweise auf der Plattform (1) nach Anspruch 26 angeordnet ist oder sind.

31. Vorrichtung nach einem der vorhergehenden Ansprüche, **gekennzeichnet durch** die Verwendung zur Aufbereitung von Biomasse für einen Fermenter einer Biogasanlage für die Erzeugung methanhaltigen Biogases aus land- oder forstwirtschaftlichen Produkten oder organischem Abfallmaterial.
